# EUROPEAN PATENT APPLICATION

(11) **EP 2 545 935 A1**
(43) Date of publication of application: **16.01.2013**
(21) Application number: 12154511.5
(22) Date of filing: 06.10.2006
(51) Int. Cl.: A61K 38/53, A61P 43/00, C12Q 1/25, A61K 38/17, C07K 14/47

(54) **HLS-5, a control sumoylation agent**

(30) Priority: 07.10.2005 US 725171 P
(62) Divisional of application: 06790340.1
(71) Applicant: Molecular Discovery Systems, North Perth, WA 6006 (AU)
(72) Inventor: Lalonde, Jean-Philippe, Australia, Western Australia 6008 (AU); Lim, Raelene, Australia, Western Australia 6111 (AU); Scaife, Robin, Australia, Western Australia 6009 (AU); Gallagher, Sam, Australia, Western Australia 6018 (AU); Klinken, S. Peter, Australia, Western Australia 6012 (AU)
(74) Representative: Gulde Hengelhaupt Ziebig & Schneider

(57) **Abstract**

The present invention relates to novel agents that are useful for controlling sumoylation. In particular, the present invention relates to a sumoylation control agent comprising: (i) a pharmaceutically-effective amount of a HLS-5 polypeptide, isoform thereof, functional fragment thereof, or pharmaceutical composition thereof; or (ii) a compound or composition capable of modulating the endogenous levels of HLS-5 or its activity; or (iii) combinations thereof.

## Description

### FIELD

The present invention relates to novel agents that are useful for controlling sumoylation. In particular, the present invention relates to a sumoylation control agent comprising either a pharmaceutically-effective amount of a HLS-5 polypeptide or functional fragment thereof or pharmaceutical composition thereof; or a compound or composition capable of modulating the endogenous levels of HLS-5 or its activity.

### BACKGROUND

Post-translational modifications of proteins have critical roles in many cellular processes because they can cause rapid changes in the functions of pre-existing individual proteins, complexes and subcellular structures. Thus, the post-translational modification of proteins is an important means of regulating protein activity, stability or localization. Recently, several small proteins have been identified with sequence similarity to ubiquitin and which modify target proteins. Some ubiquitin-like modifiers (UBLs) include SUMO (small ubiquitin-related modifier), Rubi (also called Nedd8), Apg8 and Apg12. In mammals, four members of the SUMO family have been described: SUMO-1, also known as PIC-1, sentrin or GMP1, which in humans is a 101 amino acid polypeptide; and the highly homologous polypeptides SUMO-2, SUMO-3 and SUMO-4. Although SUMO-1 shares only about 18% sequence identity to ubiquitin, both polypeptides share a common three-dimensional structure.

The pathway of protein modification by sumoylation is analogous to the well-characterized pathway of modification by ubiquitination, although a different set of enzymes is involved. SUMO is initially made as an inactive precursor. The precursor is then processed by proteolytic cleavage to yield the active modifier polypeptide with an exposed carboxy-terminal glycine residue. The exposed glycine is required for the formation of an isopeptide bond between the carboxyl terminus of SUMO and a lysine residue of the target protein. This SUMO processing reaction is catalyzed by a cysteine protease known as SUMO-activating enzyme E1 (SAE1/SAE2), SUMO-conjugating enzyme E2 (UBC9), SUMO ligases E3s, and SUMO cleaving enzymes. The E1 activating enzyme and the E2 conjugating enzyme prime SUMO for ligation to a substrate by an E3 ubiquitin ligase which confers substrate specificity (Desterro et al., 1997, FEBS Lett., 417, 297-300; Johnson et al., 1997, Embo J., 16, 5509-5519). For sumoylation RING-domain-containing proteins, in particular the protein inhibitors of activated STATs (PIAS) family were identified as E3 ligase enzymes (Johnson & Gupta, 2001, Cell, 106, 735-744. The nucleoporin RanBP2, which had been previously characterized for its function in nuclear transport, is also an E3 ligase for SUMO1 and apparently has no functional homology to RING finger motifs. RanBP2 exerts E3 ligase activity on some SUMO1 targets *in vitro* (such as Sp100) but not on all of them (indeed p53 could be sumoylated by PIAS1, but not by RanBP2), indicating substrate specificity (Pichler et al., 2002, Cell, 108, 109-120; Pichler et al., 2004, Nat Struct. Mol Biol., 11, 984-991).

PIAS proteins regulate transactivation of many transcription factors such as LEF-1 and nuclear receptors at least in part via sumoylation (kotaja et al., 2002, J Biol. Chem, 277, 17781-17788; Nishida & Yasuda, 2002, J Biol. Chem, 277, 41311-41317; Rogers et al., 2003, J Biol. Chem, 278, 30091-30097; Sachdev et al., 2001, Genes Dev., 15, 3088-3103). Recently, GATA-1 and GATA-2 were shown to be post-translationally modified by SUMO-1 and PIASy, leading to a repression of transcriptional activity (Chun et al., 2003, Circ Res, 92, 1201-1208; Collavin et al., 2004, Proc. Natl. Acad. Sci. USA, 101, 8870-8875); conversely, sumoylation increases the transactivation capacity of GATA-4 (Wang et al., 2004, J. Biol. Chem., 279(47): p49091). SUMO addition to components of the transcriptional apparatus can both activate and repress transcription. In most cases, however, SUMO modification of transcription factors leads to repression and various models have been proposed to explain this, ranging from retention in nuclear bodies to recruitment of histone deacetylases (Verger et al., 2003, EMBO Rep, 4, 137-142).

Sumoylation, as opposed to poly-ubiquitination, is a ubiquitin-like dynamic and reversible post-translational modification system which does not lead to degradation, but can affect protein interactions, subcellular localization or stability, leading to transcriptional modulation, and changes in nuclear localization (Verger *et al. ,* 2003, *supra*).

Several known SUMO substrates are important modulators of apoptosis. Apoptosis, or programmed cell death, is involved in the development and homeostasis of multicellular organisms. Alterations in the normal process of apoptosis occur in various pathological conditions, including cancer, autoimmune diseases, inflammatory conditions, degenerative syndromes and infectious diseases.

Viruses have multifunctional proteins that can target essential biochemical pathways within host cells. Some viral proteins have also been identified as substrates for SUMO modification as well as altering the sumoylation status of host cell proteins (Wilson & Rangasamy, 2001, Virus Res., 81, 17-27.). Viruses such as human papillomavirus, cytomegalovirus, adenovirus and the herpesviruses, may utilize a cell's SUMO for their own deleterious purposes. In some cases, sumoylation of viral proteins targets them to the nucleus so that they can take over the cell's replication machinery, thus allowing viral reproduction. Interestingly, an adenoviral protein, Gam1, a strong global transcriptional activator of both viral and cellular genes also inhibits the SUMO pathway by interfering with the activity of E1 heterodimer (SAE1/SAE2), leading to the accumulation of SUMO-unmodified substrates (Boggio et al., 2004, Mol Cell, 16, 549-561).

The finding that SUMO substrates include many proteins with important roles in regulating cell proliferation and differentiation suggests that, like ubiquitination, altered SUMOylation may contribute to disease onset or progression. In fact, recent data suggest that several diseases including pathogenic infection, cancer, and neurodegenerative disorders are associated with alterations in SUMOylation. The cellular SUMOylation system is exploited by several viral and bacterial pathogens to favor replication and infection (Wilson & Rangasamy, 2001, supra). One striking example is the finding that the YopJ protein of *Yersinia pestis,* the bacterial agent for the Black Plague, encodes a SUMO protease homolog whose activity contributes to inhibition of the host immune response (Orth et al., 2000, Science, 290, 1594-1597). The observation that the activity and/or localization of several tumor suppressors and oncogenes is regulated by SUMO modification raises the possibility that alterations in the SUMO conjugation machinery or alterations in SUMO modification of specific substrates may contribute to cancer. Although this hypothesis requires further investigation, it is interesting to note that in acute promyelocytic leukemia, the PML-RAR fusion protein is not SUMO modified whereas arsenic trioxide, an effective treatment for this disorder, restores SUMOylation of the fusion protein (Miller et al., 1998, Blood, 92, 4308-4316). SUMO has been shown to localize to intranuclear inclusions that characterize several neurodegenerative diseases including Huntington's, Parkinson and Neuronal intranuclear inclusion disease (Pountney et al., 2003, Exp Neurol, 184, 436-446*;* Terashima et al., 2002; Neuroreport, 13, 2359-2364; Ueda et al., 2002, Biochem Biophys Res Commun, 293, 307-313). Over-expression of SUMO-2 influences processing of the α-amyloid precursor protein, an event implicated in the onset of Alzheimer's disease, and genetic manipulation of the SUMO conjugation pathway in Drosophila indicates that SUMOylation contributes to neurodegeneration in at least two polyglutamine repeat diseases, spinal and bulbar muscular atrophy (SBMA) and Huntington's (Chan et al., 2002, Hum Mol Genet, 11, 2895-2904; Li et al., 2003, Proc Natl Acad Sci USA, 100, 259-264; Steffan et al., 2004, Science, 304, 100-104).

Accordingly, in addition to the possibility of revealing new insights into the mechanisms that regulate gene expression, genome maintenance, and signal transduction, an increased understanding of the regulation and function of the post-translational modifier SUMO and controlling its addition to target proteins may provide new targets for therapeutic intervention in several human diseases.

### SUMMARY

The present inventors have found that HLS-5 is capable of affecting sumoylation. In some instances, HLS-5 inhibits some molecules from becoming sumoylated by affecting members of the sumoylation pathway and/or by competing for substrates, for example, PIAS1, SUMO1 and Ubc9. While in other instances, HLS-5 increases sumoylation of some proteins. The present inventors have also shown that HLS-5 can have a global effect on sumoylation i.e. it affects sumoylation of a large variety of proteins.

Accordingly, in a first aspect the present invention provides a sumoylation control agent comprising:
(i) a pharmaceutically-effective amount of a HLS-5 polypeptide, isoform thereof, functional fragment thereof or pharmaceutical composition thereof; or
(ii) a compound or composition capable of modulating the endogenous levels of HLS-5 or its activity; or
(iii)combinations thereof.

In some embodiments, the HLS-5 polypeptide will comprise the sequence set out in SEQ ID NO:2, SEQ ID NO:4 or SEQ ID NO:6 or a polypeptide substantially homologous thereto, or a functional fragment thereof.

It is to be clearly understood that the HLS-5 polypeptide of the present invention also includes polypeptide analogues, including but not limited to the following:
1. HLS-5 polypeptide in which one or more amino acids is replaced by its corresponding D-amino acid. The skilled person will be aware that retro-inverso amino acid sequences can be synthesised by standard methods. See for example Chorev and Goodman, 1993, ACC Chem Res, 26: 266-273;
2. Peptidomimetic compounds of HLS-5, in which the peptide bond is replaced by a structure more resistant to metabolic degradation. See, for example, Olson et al, 1993, J. Med. Chem. , 36, p3039-3049.
3. HLS-5 polypeptide in which individual amino acids are replaced by analogous structures, for example *gem-*diaminoalkyl groups or alkylmalonyl groups, with or without modified termini or alkyl, acyl or amine substitutions to modify their charge.

The use of such alternative structures can provide significantly longer half-life in the body, since they are more resistant to breakdown under physiological conditions.

Methods for combinatorial synthesis of polypeptide analogues and for screening of polypeptides and polypeptide analogues are well known in the art (see, for example, Gallop et al., 1994, J. Med. Chem., 37, p1233-1251). It is particularly contemplated that the HLS-5 polypeptides of the invention are useful as templates for design and synthesis of compounds of improved activity, stability and bioavailability.

Preferably where amino acid substitution is used, the substitution is conservative, *i.e*., an amino acid is replaced by one of similar size and with similar charge properties.

In some embodiments, the HLS-5 polypeptide will be expressed *in vivo* from a vector comprising a polynucleotide encoding HLS-5. In some embodiments, the HLS-5 polynucleotide will be selected from one or more of the group consisting of:
(a) polynucleotides comprising the nucleotide sequence set out in SEQ ID NO:1, SEQ ID NO:3 or SEQ ID NO:5, or a functional fragment thereof;
(b) polynucleotides comprising a nucleotide sequence capable of hybridizing selectively to the nucleotide sequence set out in SEQ ID NO:1, SEQ ID NO:3 or SEQ ID NO:5, or a functional fragment thereof;
(c) polynucleotides comprising a polynucleotide sequence which is degenerate as a result of the genetic code to the polynucleotides defined in (a) or (b); and
(d) polynucleotides complementary to the polynucleotides of (a) or (b).

The present invention also provides a vector comprising a HLS-5 polynucleotide, for example an expression vector comprising a HLS-5 polynucleotide, operably linked to regulatory sequences capable of directing expression of said polynucleotide in a host cell.

Accordingly, in a second aspect, the present invention provides a sumoylation control agent comprising a vector comprising a HLS-5 polynucleotide, operably linked to regulatory sequences capable of directing expression of said polynucleotide in a host cell.

In a third aspect the present invention provides a method of regulating sumoylation in vivo comprising the step of administering to a subject in need thereof:
(i) a pharmaceutically-effective amount of a HLS-5 polypeptide, isoform thereof, functional fragment thereof or pharmaceutical composition thereof; or
(ii) a compound or composition capable of modulating the endogenous levels of HLS-5 or its activity; or
(iii)combinations thereof.

In some embodiments, the sumoylation control agent will negatively control sumoylation i.e. directly or indirectly prevent sumoylation and/or reverse sumoylation by a desumoylation process. In yet other embodiments, the sumoylation control agent will positively control sumoylation i.e. directly or indirectly bring about sumoylation.

In a fourth aspect the present invention provides a method of regulating sumoylation *in vitro* comprising the step of administering to cells:
(i) a pharmaceutically-effective amount of a HLS-5 polypeptide, isoform thereof, functional fragment thereof or pharmaceutical composition thereof; or
(ii) a compound or composition capable of modulating the endogenous levels of HLS-5 or its activity; or
(iii)combinations thereof.

In a fifth aspect the present invention provides a method for treating or preventing a condition comprising the step of administering to a subject:
(i) a pharmaceutically-effective amount of a HLS-5 polypeptide, isoform thereof, functional fragment thereof or pharmaceutical composition thereof; or
(ii) a compound or composition capable of modulating the endogenous levels of HLS-5 or its activity; or
(iii)combinations thereof.

In some embodiments the condition will be directly affected by or controlled by sumoylation. In other embodiments, the condition will not be directly affected by or controlled by sumoylation; however, the administration of the sumoylation control agent improves, alleviates or treats the condition by controlling the sumoylation of polypeptides associated with or affected by the condition.

The sumoylation control agents of the invention may be administered by any suitable route, and the person skilled in the art will readily be able to determine the most suitable route and dose for the condition to be treated. Dosage will be at the discretion of the attendant physician or veterinarian, and will depend on the nature and state of the condition to be treated, the age and general state of health of the subject to be treated, the route of administration, and any previous treatment which may have been administered.

The sumoylation control agent may be administered in the form of a composition further comprising a pharmaceutically acceptable carrier. This will usually comprise at least one excipient, for example selected from the group consisting of sterile water, sodium phosphate, mannitol, sorbitol, sodium chloride, and any combination thereof.

Methods and pharmaceutical carriers for preparation of pharmaceutical compositions are well known in the art, as set out in textbooks such as Remington's Pharmaceutical Sciences, 20th Edition, Williams & Wilkins, Pennsylvania, USA.

The carrier or diluent, and other excipients, will depend on the route of administration, and again the person skilled in the art will readily be able to determine the most suitable formulation for each particular case.

The subject may be a human, or may be a domestic, companion or zoo animal. While it is particularly contemplated that the sumoylation control agents of the invention are suitable for use in medical treatment of humans, they are also applicable to veterinary treatment, including treatment of companion animals such as dogs and cats, and domestic animals such as horses, cattle and sheep, or zoo animals such as non-human primates, felids, canids, bovids, and ungulates.

In a sixth aspect the present invention provides an assay for identifying a modifier of the sumoylation activity of an HLS-5 polypeptide, isoform thereof, or functional fragment thereof, , comprising:
(i) providing a sumoylation-conjugating system including a substrate polypeptide and sumo, under conditions which promote sumoylation of the substrate polypeptide by sumo;
(ii) contacting the sumoylation-conjugating system with HLS-5;
(iii) measuring the level of sumoylation of the substrate polypeptide in the presence of the HLS-5;
(iv) contacting the sumoylation-conjugating system with a candidate agent;
(v) measuring the level of sumoylation of the substrate polypeptide in the presence of the candidate agent; and
(iv) comparing the measured level of sumoylation in the presence of the candidate agent with sumoylation of the substrate polypeptide in the presence of HLS-5 and/or absence of the candidate agent, wherein a statistically significant alteration in sumoylation of the substrate polypeptide in the presence of the candidate agent is indicative of a modifier of the sumoylation activity of the HLS-5 polypeptide, isoform thereof, or functional fragment thereof.

In a seventh aspect the present invention provides an assay for identifying a modifier of the endogenous level of HLS-5 polypeptide, isoform thereof, or functional fragment thereof, comprising:
(i) measuring the level of HLS-5 in a biological sample from a subject;
(ii) contacting the biological sample with a candidate agent;
(iii) measuring the level of HLS-5 in the contacted sample of (ii); and
(iv) comparing the measured level of HLS-5 of (i) and (ii), wherein a statistically significant difference in the level is indicative of a modifier of the endogenous level of HLS polypeptide, isoform thereof, or functional fragment thereof.

In an eighth aspect the present invention provides a method of determining the suitability of HLS-5 treatment of a condition, comprising:
(i) providing a biological sample from a test subject;
(ii) measuring the level of sumoylation of a substrate polypeptide in the biological sample; and
(iii) contacting the biological sample with HLS-5 and measuring the level of sumoylation of the substrate polypeptide and comparing the measured level of sumoylation in the presence of the HLS-5 with sumoylation of the substrate polypeptide in the absence of HLS-5, wherein a statistically significant difference in sumoylation of the substrate polypeptide in the presence of the HLS-5 is indicative of the suitability of HLS-5 treatment of the condition.

### BRIEF DESCRIPTION OF THE FIGURES

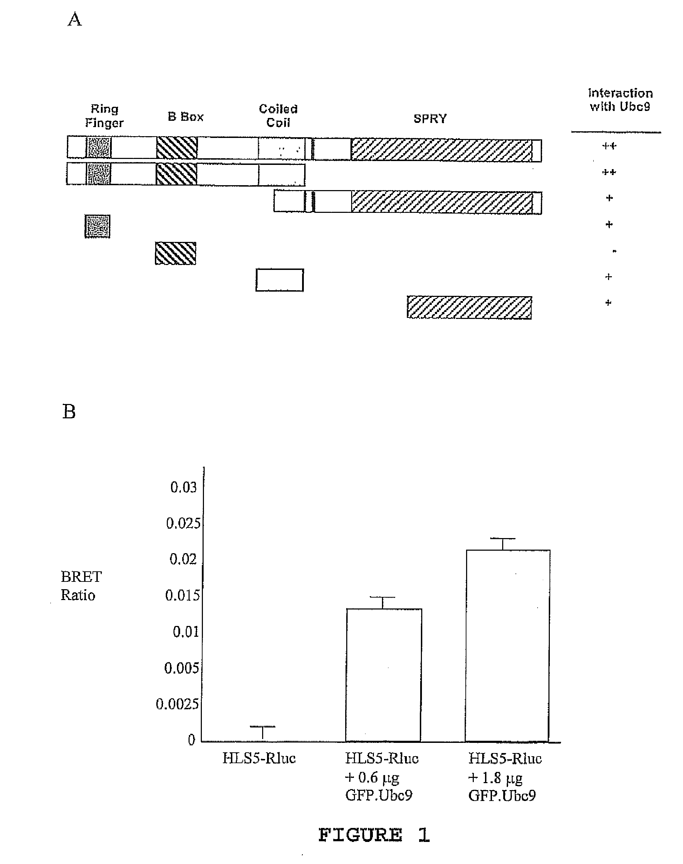

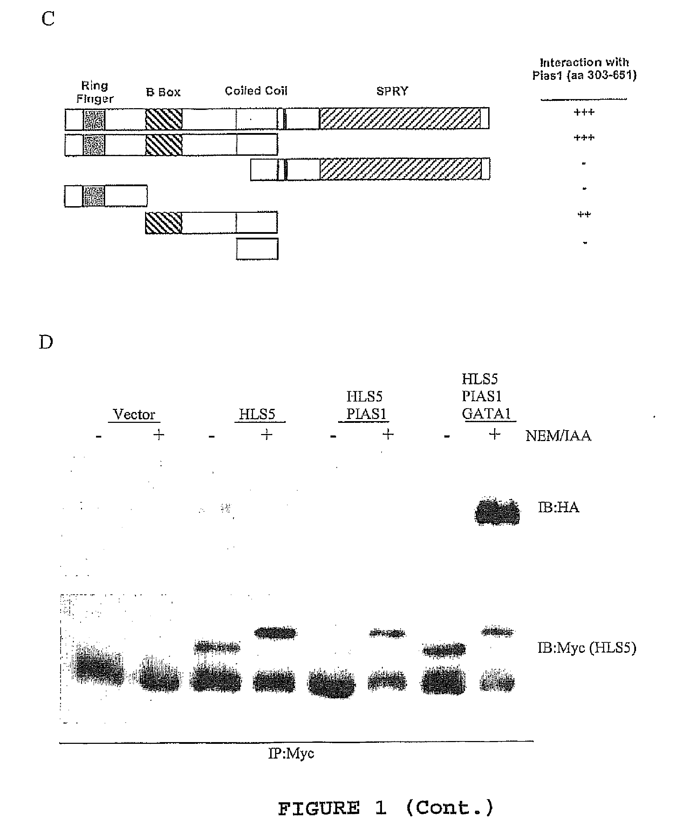

Figure 1 shows that HLS-5 binds Ubc9 and Pias1. (A) Full length Ubc9 was examined for binding to various HLS-5 constructs as shown using yeast two-hybrid. Binding was measured by filter assay for β-galactosidase activity. (B) BRET assay of COS7 cells expressing HLS-5.Rluc fusion protein with increasing amounts of GFP-Ubc9 fusion protein. Results presented as BRET ratio. (C) PIAS1 (aa 303-651) fragment isolated from the Y2H screen was assessed for binding to various HLS-5 constructs as shown using yeast two-hybrid. Binding was measured by filter assay for β-galactosidase activity. (D) Lysates from COS7 cells transfected with Myc-tagged HLS-5, HA-tagged PIAS1 and/or GATA-1 were immunoprecipitated (*IP*) with anti-Myc antibodies and immunoblotted (*IB*) with antibodies to HA or Myc in the presence and/or absence of NEM/IAA.

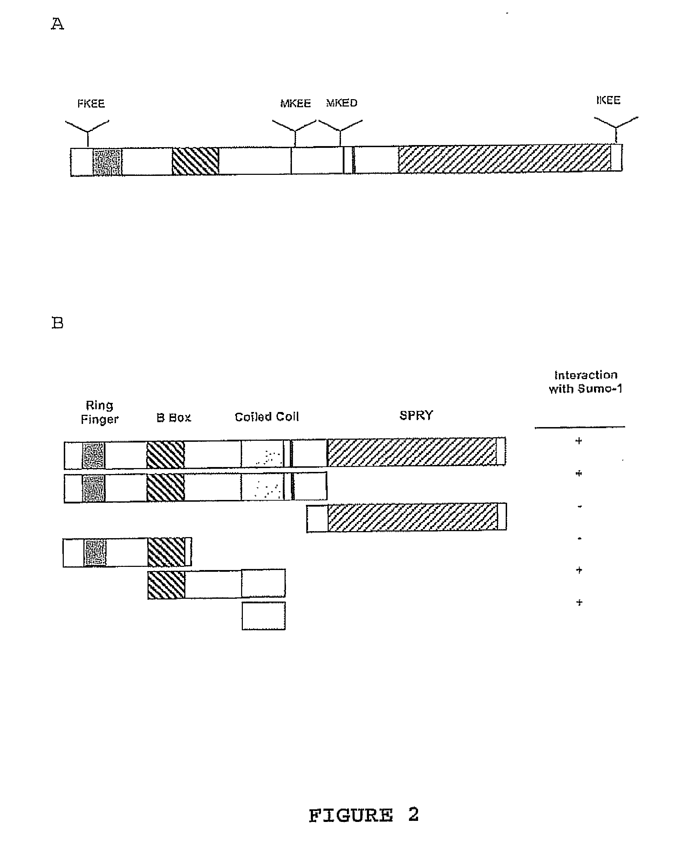

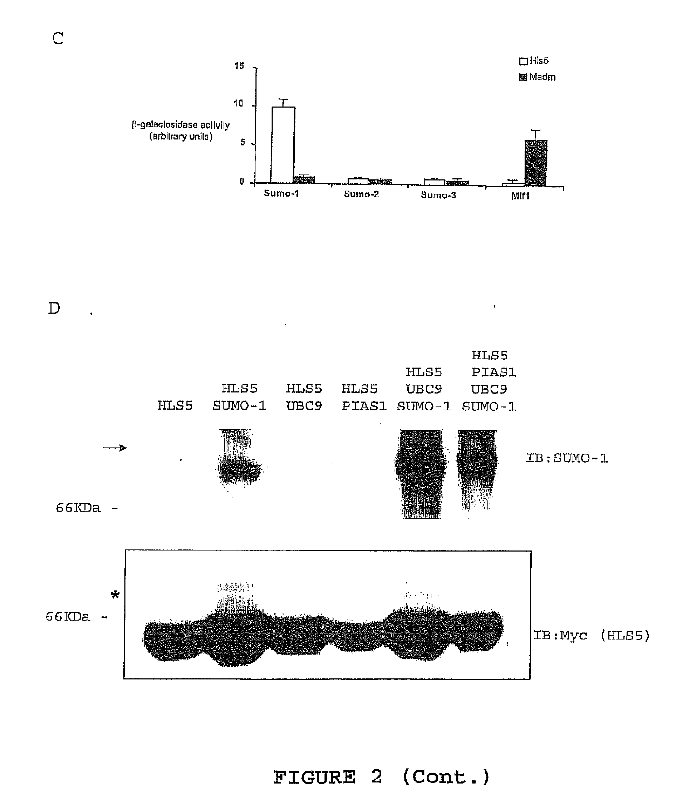

Figure 2 shows that HLS-5 binds SUMO-1 and is SUMOylated *in vivo.* (A) Potential sumoylation sites of HLS-5 are shown. (B) Binding of Hls-5 deletion mutants to full length Sumo-1 was examined in the yeast system by filter assay for β-galactosidase activity. (C) Interaction between Hls-5 and Sumo-1, 2 or 3 was examined using yeast two-hybrid. Binding was assessed using a liquid assay to measure β-galactosidase activity. Samples were measured in triplicate and shown is the mean ± standard deviation. (D) Lysates from COS7 cells transfected with Myc-tagged Hls-5, and/or HA-tagged Sumo-1, HA-tagged UBC9, HA-tagged PIAS1 were immunoblotted (*IB*) with antibodies to Sumo-1 or Myc.

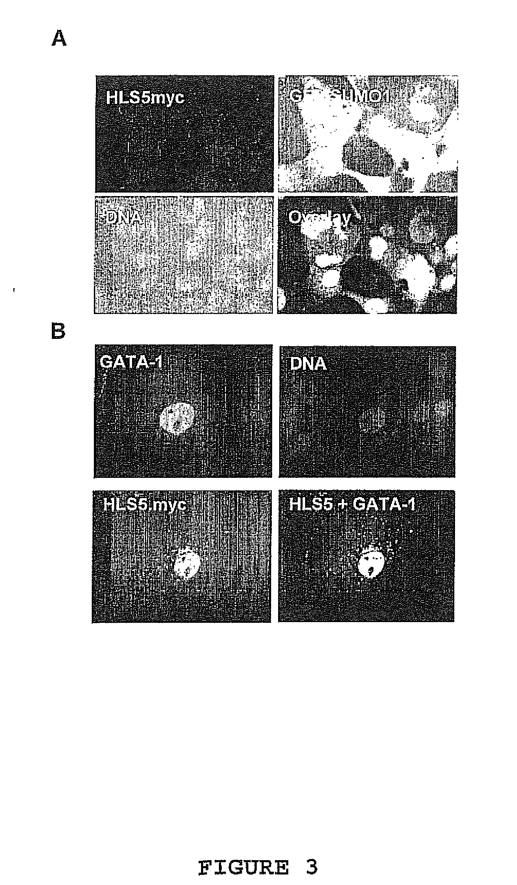

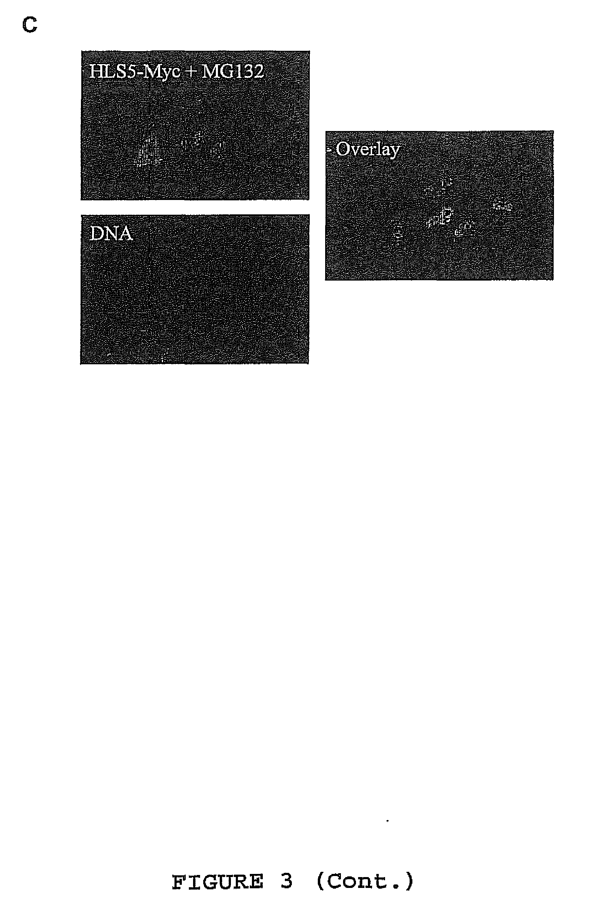

Figure 3 shows that GATA-1, MG-132 and SUMO-1 alter HLS-5 localisation. (A) Confocal microscopy of COS7 cells co-expressing Myc-tagged HLS-5 and GFP-SUMO-1. HLS-5 was detected with anti-Myc antibodies in conjunction with red secondary antibodies and SUMO-1 detected by green fluorescent. (B) Confocal microscopy of COS7 cells co-expressing Myc-tagged HLS-5 and GATA-1. GATA-1 was detected with an anti-GATA-1 antibody and HLS-5 was detected with anti-Myc antibodies in conjunction with red and green fluorescent secondary antibodies respectively. (C) Confocal microscopy of COS7 cells co-expressing Myc-tagged HLS-5 in the presence of MG132. HLS-5 was detected with anti-Myc antibodies in conjunction with red fluorescent secondary antibodies

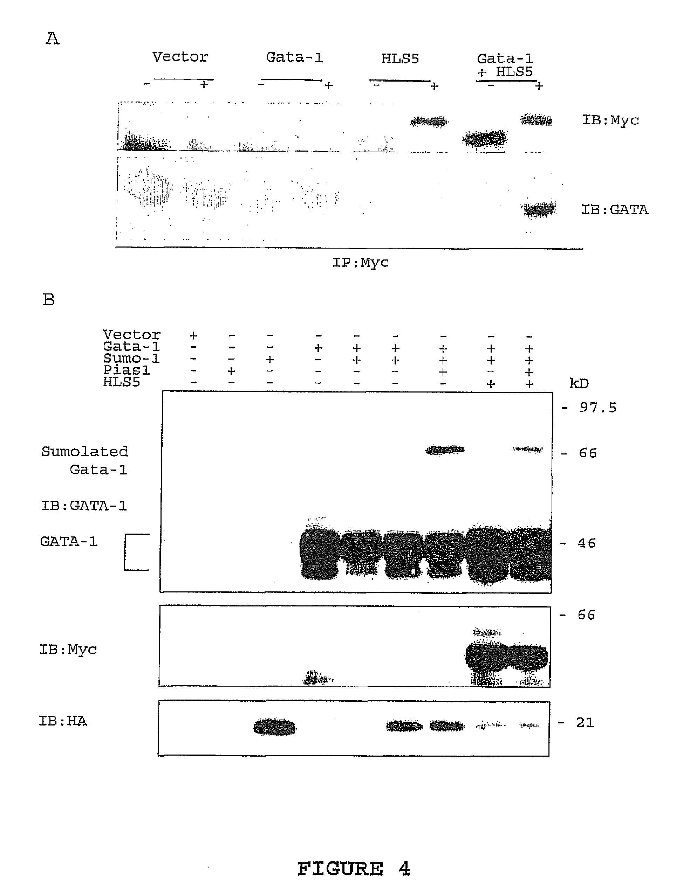

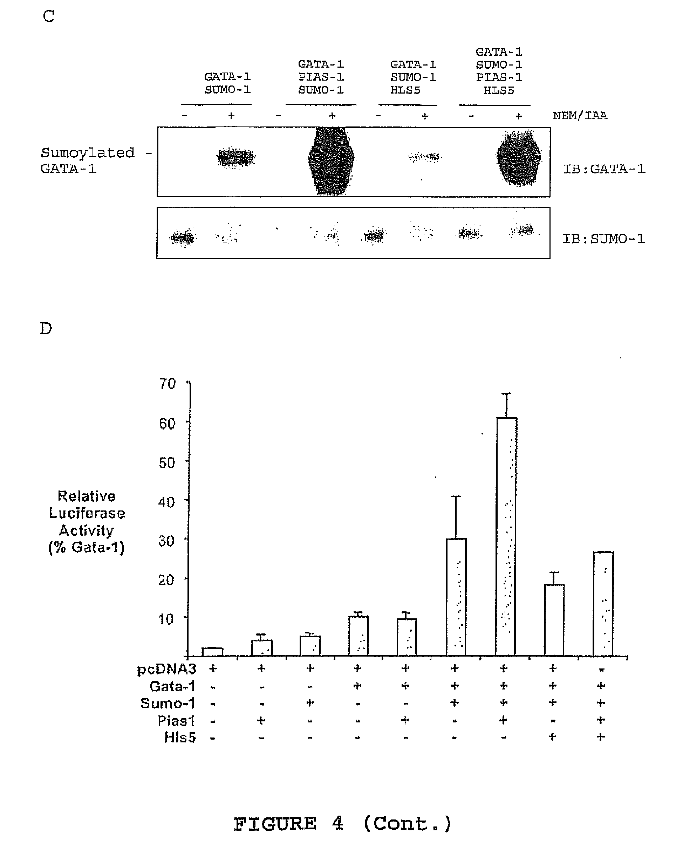

Figure 4 shows GATA-1 interacts with HLS-5 and that the interaction is dependant on isopeptidase inhibitors. HLS-5 also inhibits the general GATA-1 SUMOylation and the PIAS1 induced SUMOylation as well as repress the transcriptional activity of GATA-1.(A) Lysates from COS7 cells transfected with Myc-tagged Hls5 and/or Gata-1 were immunoprecipitated (*IP*) with anti-Myc antibodies and immunoblotted (*IB*) with antibodies to GATA-1 or Myc. (B and C) Lysates from COS7 cells transfected with Myc-tagged HLS-5 and/or GATA-1, and/or HA-tagged Sumo-1, and/or HA-tagged PIAS1 were immunoblotted (*IB*) with antibodies to GATA-1, HA or Myc D) COS7 cells were transfected as shown in the presence of pGL3-M1α firefly luciferase reporter construct and pRN-CMV renilla luciferase construct. Relative GATA-1 activity was determined by dividing firefly activity with renilla activity. Amount of DNA used was 200ng per transfection (+) unless otherwise shown. Samples were performed in duplicate and shown is the mean ± range. Data shown are representative of three independent experiments.

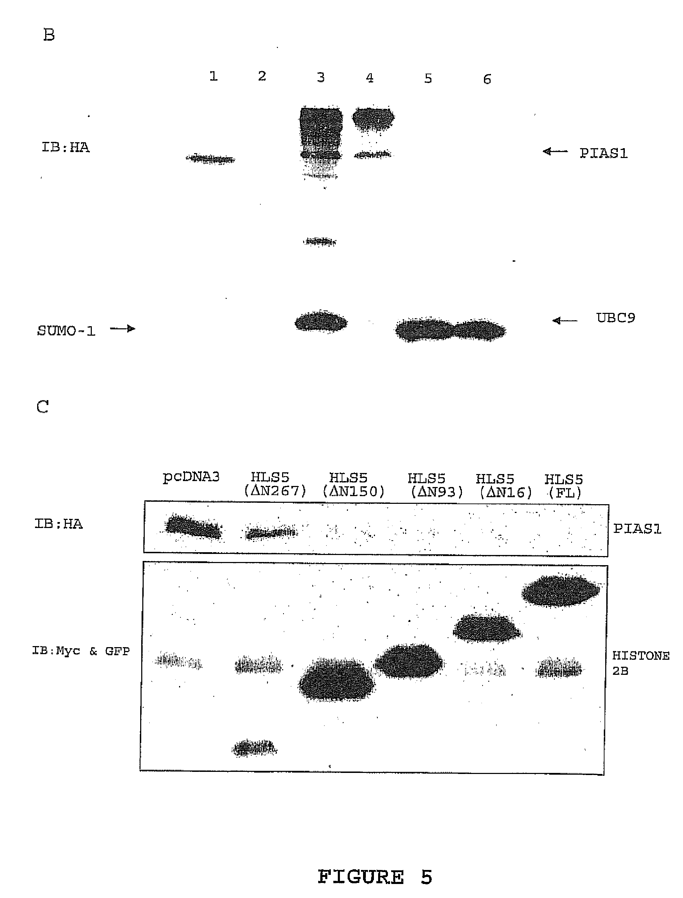

Figure 5 shows localisation and that HLS-5 leads to the degradation of UBC9 and PIAS1 through its Coiled Coil domain. (A) Hela cells co-expressing Myc-tagged HLS-5 and HA-tagged PIAS1. PIAS1 was detected with an anti-HA antibody and HLS-5 was detected as a GFP fusion protein in conjunction with red and green fluorescent secondary antibodies respectively. (B) COS7 cells transfected with GFP-tagged HLS-5 and/or HA-tagged PIAS1, UBC9 or SUMO-1 and immunoblotted (*IB*) with antibodies to HA. (C) Lysates from COS7 cells transfected with Different Myc-tagged HLS-5 deletion constructs and HA-tagged-PIAS1, and GFP-tagged Histone 2B as a transfection/Loading control. Samples were immunoblotted (*IB*) with antibodies to GFP, HA and Myc.

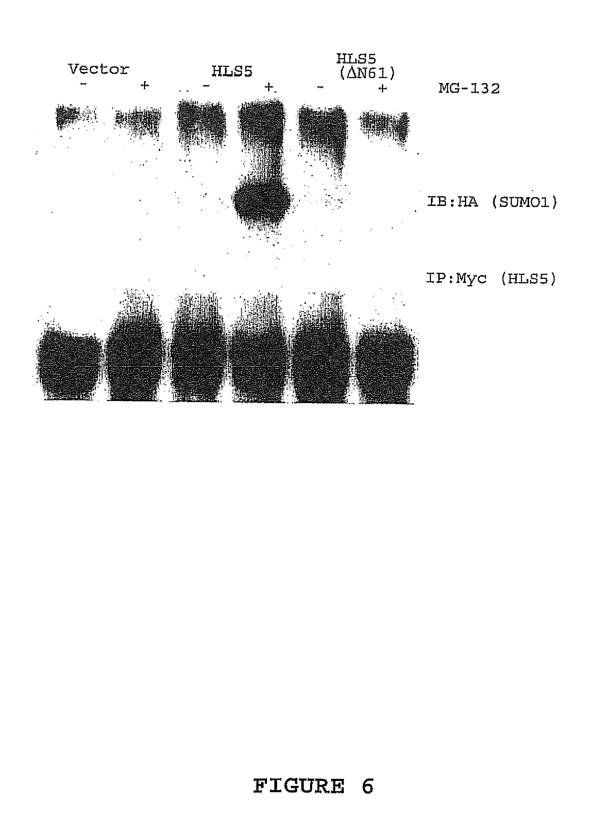

Figure 6 shows that HLS-5 expression in the presence of MG132 can increase SUMOlation of a substrate which co-immunoprecipitates with Myc-tagged HLS-5. (A) COS7 cells co-expressing Myc-tagged HLS-5 or a mutant form of HLS5 lacking its Ring finger (ΔN61) and HA-tagged SUMO1 in the presence or absence of MG132. SUMOylated substrate was detected with an anti-HA antibody and HLS-5 was immunoprecipitated by using a Myc antibodies respectively.

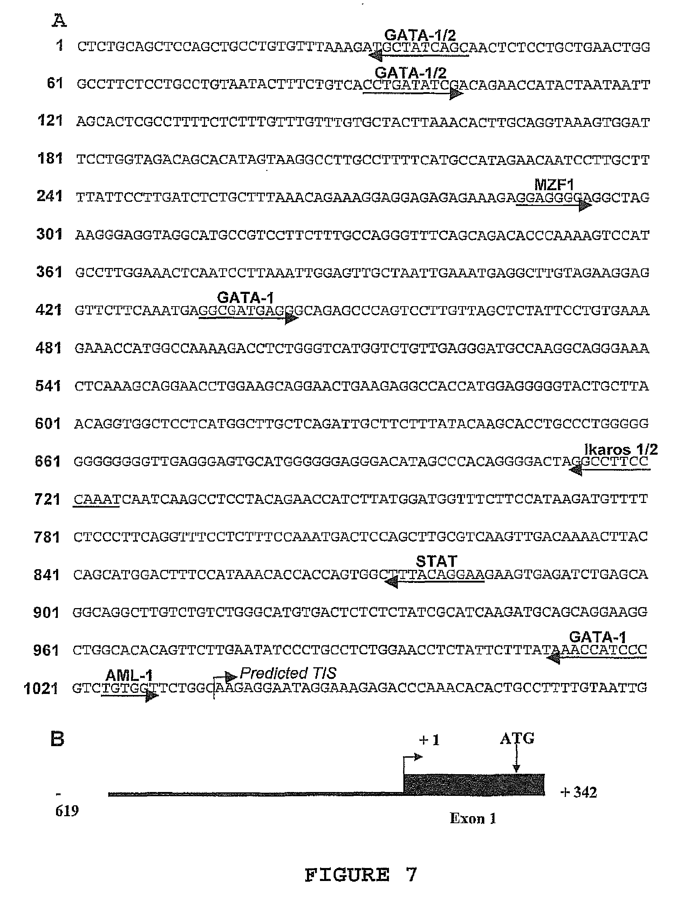

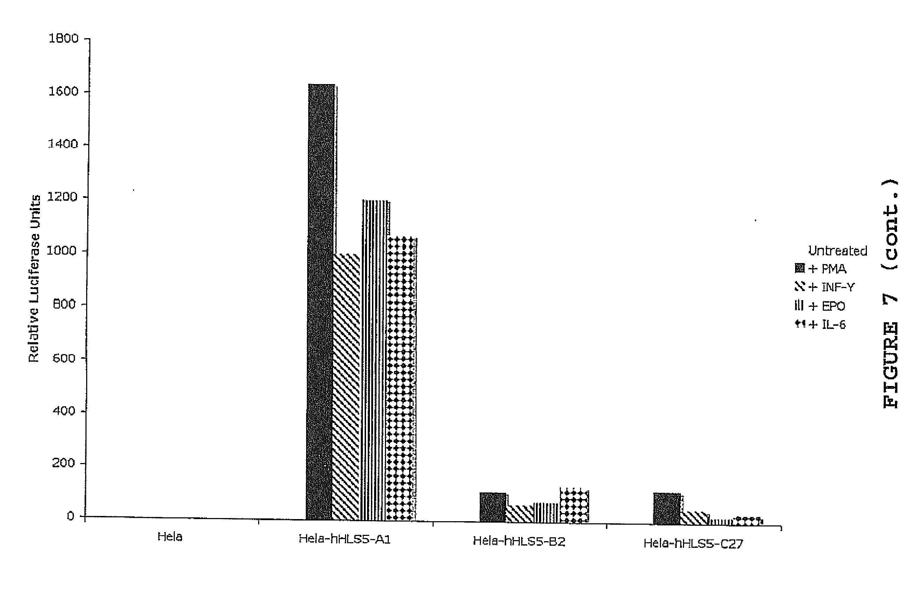

Figure 7 shows generation of luciferase expressing HLS-5 promoter stable cell lines and development of primary assay for high throughput screening. (A) HLS5 5' UTR protential transcription binding site. (B) The structure of the HLS5 promoter subcloned upstream of the luciferase reporter vector. (C) Parental HeLa cells and HLS-5 promoter expressing cell lines were tested for their response to various inducing compounds.

### DETAILED DESCRIPTION OF PREFERRED EMBODIMENTS

Before describing the present invention in detail, it is to be understood that this invention is not limited to particularly exemplified HLS-5 sequences, expression techniques or methods and may, of course, vary. It is also to be understood that the terminology used herein is for the purpose of describing particular embodiments of the invention only, and is not intended to be limiting which will be limited only by the appended claims.

All publications, patents and patent applications cited herein, whether *supra* or *infra*, are hereby incorporated by reference in their entirety. However, publications mentioned herein are cited for the purpose of describing and disclosing the protocols and reagents which are reported in the publications and which might be used in connection with the invention. Nothing herein is to be construed as an admission that the invention is not entitled to antedate such disclosure by virtue of prior invention.

The practice of the present invention will employ, unless otherwise indicated, conventional techniques of cell biology, cell culture, molecular biology, recombinant DNA, pharmacology and immunology, which are within the skill of the art. Such techniques are described in the literature. See, for example, Bailey & Ollis, 1986, "Biochemical Engineering Fundamentals", 2nd Ed., McGraw-Hill, Toronto; Coligan et al., 1999, "Current protocols in Protein Science" Volume I and II (John Wiley & Sons Inc.); "DNA Cloning: A Practical Approach", Volumes I and II (Glover ed., 1985); Handbook of Experimental Immunology, Volumes I-IV (Weir & Blackwell, eds., 1986); Immunochemical Methods in Cell and Molecular Biology (Mayer and Walker, eds., Academic Press, London, 1987), Methods in Enzymology, Vols. 154 and 155 (Wu et al. eds. 1987); "Molecular Cloning: A Laboratory Manual", 2nd Ed., (ed. by Sambrook, Fritsch and Maniatis) (Cold Spring Harbor Laboratory Press: 1989); "Nucleic Acid Hybridization", (Hames & Higgins eds. 1984); "Oligonucleotide Synthesis" (Gait ed., 1984); Remington's Pharmaceutical Sciences, 17th Edition, Mack Publishing Company, Easton, Pennsylvania, USA.; "The Merck Index", 12th Edition (1996), Therapeutic Category and Biological Activity Index; and "Transcription & Translation", (Hames & Higgins eds. 1984).

It must be noted that as used herein and in the appended claims, the singular forms "a," "an," and "the" include plural reference unless the context clearly dictates otherwise. Thus, for example, a reference to "a nucleic acid molecule" includes a plurality of such molecules, and a reference to "an agent" is a reference to one or more agents, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

The present invention encompasses the following aspects: preparation of a HLS-5 sumoylation control agent of the present invention; preparation of the polynucleotide encoding said HLS-5 polypeptide or a recombinant vector carrying and expressing said polynucleotide; transformants carrying said vector; methods of producing said transformants; methods of detecting the HLS-5 polypeptide; methods of detecting the mRNA or polynucleotide encoding said HLS-5 polypeptide; and methods of treating conditions caused by or exacerbated by unregulated sumoylation are explained below.

In the description that follows, if there is no instruction, it will be appreciated that techniques such as gene recombinant techniques, production of recombinant polypeptides in animal cells, insect cells, yeast and *Escherichia coli,* molecular-biological methods, methods of separation and purification of expressed HLS-5 polypeptides, assays and immunological methods, are well-known in this field and any such technique may be adopted.

In its broadest aspect the present invention provides a sumoylation control agent comprising a pharmaceutically-effective amount of a HLS-5 polypeptide, isoform thereof or functional fragment thereof.

The term "sumoylation control agent" as used herein refers to a compound or composition of matter that is capable of affecting directly or indirectly the sumoylation of a protein. As described *supra*, sumoylation is the post-translational modification of a protein, wherein "SUMO-1', a ubiquitin-like protein, is ligated to a lysine residue in a target protein.

In some embodiments of the present invention the "sumoylation control agent" is HLS-5. HLS-5 is a member of the RING finger B-box Coiled-coil (RBCC) protein family (Lalonde et al., 2004, J Biol Chem, 279, 8181-8189). This group of molecules is also referred to as the tripartite motif family (TRIM) of proteins, because of the characteristic domain architecture that is conserved amongst higher eukaryotes (Reymond et al., 2001, Embo J, 20, 2140-2151). Sequence analysis of the mouse and human genomes has identified a diverse array of RBCC proteins, many with unknown functions (Reymond *et al.,* 2001, *supra*)*.* Several RBCC family members, including PML, TIF1α and Rfp, are mutated in human cancer, implicating RBCC proteins as crucial regulators of cell growth and differentiation (de The et al 1991, Cell, 66:675-684). HLS-5 maps to chromosome 8p21, a region frequently deleted in a variety of tumours, and enforced expression of the gene in HeLa cells reduced cell growth, clonogenicity and tumorigenicity, suggesting that it is a tumour suppressor gene (Lalonde *et al.,* 2004, *supra).* Recent studies have demonstrated that some RBCC members regulate the activity, or steady-state levels, of partner proteins by influencing subcellular localization or post-translational modifications (Diamonti et al., 2002, Proc Natl Acad Sci USA, 99, 2866-2871; Pearson et al., 2000, Nature, 406, 207-210; Urano et al., 2002, Nature, 417, 871-875).

HLS-5 was originally identified as a gene markedly up-regulated during an erythroid to myeloid lineage switch of the J2E erythroid cell line (Klinken et al., 1988, Proc. Natl. Acad. Sci., USA, 85, 8506-8510; Lalonde *et al.,* 2004, *supra).* The myeloid variants displayed a monoblastoid morphology, did not respond to erythropoietin (EPO) and had reduced expression of erythroid-specific transcription factors, including GATA-1 and EKLF (Keil et al., 1995, Cell Growth Differ., 6, 439-448; Williams et al., 1999, Embo J., 18, 5559-5566). Significantly, HLS-5 was isolated independently as a gene induced during macrophage colony stimulating factor - initiated maturation of myeloid cells (Kimura et al., 2003, J Biol. Chem., 278, 25046-25054).

Therefore, in some embodiments of the present invention the "sumoylation control agent" comprises an isolated full-length HLS-5 polypeptide. The term "polypeptide" refers to a polymer of amino acids and its equivalent and does not refer to a specific length of the product; thus, peptides, oligopeptides and proteins are included within the definition of a polypeptide. This term also does not refer to, or exclude modifications of the polypeptide, for example, glycosylations, acetylations, phosphorylations, and the like. Included within the definition are, for example, polypeptides containing one or more analogs of an amino acid (including, for example, natural amino acids, etc.), polypeptides with substituted linkages as well as other modifications known in the art, both naturally and non-naturally occurring.

Full length HLS-5 polypeptides used in the present invention have about 500 amino acids, encode a tumor suppressor factor in an animal, particularly a mammal, and include allelic variants or homologues. Full length HLS-5 polypeptides also typically comprise a Ring finger motif, a B box, a coiled-coil motif and a SPRY motif. HLS-5 polypeptides used in the invention also include fragments and derivatives of full length HLS-5 polypeptides, particularly fragments or derivatives having substantially the same biological activity. The polypeptides can be prepared by recombinant or chemical synthetic methods. In some embodiments, the HLS-5 polypeptides include those comprising the amino acid sequence of SEQ ID NO:2, SEQ ID NO:4 or SEQ ID NO:6, or allelic variants or homologues, including fragments, thereof. In further embodiments, the HLS-5 polypeptides consist essentially of amino acids 12 to 504 of the amino acid sequence shown as SEQ ID NO:4 or allelic variants, homologues or fragments, thereof.

In the context of the present invention, a homologous sequence is taken to include an amino acid sequence which is at least 60, 70, 80 or 90% identical, preferably at least 95 or 98% identical at the amino acid level over at least 20, 50, 100, 200, 300 or 400 amino acids with the amino acid sequences set out in SEQ ID NO:2, SEQ ID NO:4, SEQ ID NO:6 or SEQ ID NO:8. In particular, homology should typically be considered with respect to those regions of the sequence known to be essential for the function of the protein rather than non-essential neighbouring sequences. Thus, for example, homology comparisons are preferably made over regions corresponding to the Ring finger, B box, coiled coil and/or SPRY domains of the HLS-5 amino acid sequence set out in SEQ ID NO:2, SEQ ID NO:4 or SEQ ID NO:8. The ring finger corresponds to approximately amino acids 36 to 75 of SEQ ID NO:2. The B box corresponds to approximately amino acids 111 to 152 of SEQ ID NO:2. The coiled coil corresponds to approximately amino acids 219 to 266 of SEQ ID NO:2.

The SPRY domain corresponds to approximately amino acids 368 to 507 of SEQ ID NO:2. In some embodiments, polypeptides of the invention comprise a contiguous sequence having greater than 50, 60 or 70% homology, more preferably greater than 80 or 90% homology, to one or more of amino acids 111 to 152, 219 to 266 or 368 to 507 of SEQ ID NO:2 or the corresponding regions of SEQ ID NO:4 or SEQ ID NO:6.

In some embodiments, polypeptides may alternatively or in addition comprise a contiguous sequence having greater than 80 or 90% homology, to amino acids 36 to 75 of SEQ ID NO:2 or the corresponding region of SEQ ID NO:4 or SEQ ID NO:6. Other polypeptides comprise a contiguous sequence having greater than 40, 50, 60, or 70% homology, more preferably greater than 80 or 90% homology to amino acids 1 to 35, 76 to 110, 153 to 218 and/or 267 to 367 of SEQ ID NO:2 or the corresponding regions of SEQ ID NO:4 or SEQ ID NO:6. Although homology can also be considered in terms of similarity (i.e. amino acid residues having similar chemical properties/functions), in the context of the present invention it is preferred to express homology in terms of sequence identity. The terms "substantial homology" or "substantial identity", when referring to polypeptides, indicate that the polypeptide or protein in question exhibits at least about 70% identity with an entire naturally-occurring protein or a portion thereof, usually at least about 80% identity, and preferably at least about 90 or 95% identity.

Homology comparisons can be conducted by eye, or more usually, with the aid of readily available sequence comparison programs. These commercially available computer programs can calculate percentage homology between two or more sequences.

Percent (%) homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relative short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible-reflecting higher relatedness between the two compared sequences-will achieve a higher score than one with many gaps. "Affine gap costs" are typically used that charge a relative high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, USA; Devereux et al., 1984, Nucleic Acids Research, 12: 387). Examples of other software than can perform sequence comparisons include, but are not limited to, the BLAST package (see, Ausubel *et al., supra*), FASTA (Atschul et al., 1990, J. Mol. Biol., 403-410) and the GENEWORKS suite of comparison tools. Both BLAST and FASTA are available for offline and online searching (see Ausubel *et al., supra,* pages 7-58 to 760). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix-the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

HLS-5 polypeptide homologues include those having the amino acid sequences, wherein one or more of the amino acids are substituted with another amino acid which substitutions do not substantially alter the biological activity of the molecule.

An HLS-5 polypeptide homologue according to the invention preferably has 80% or greater amino acid sequence identity to the human HLS-5 polypeptide amino acid sequence set out in SEQ ID NO:4 or SEQ ID NO:6. Examples of HLS-5 polypeptide homologues within the scope of the invention include the amino acid sequence of SEQ ID NO:4 or SEQ ID NO:6 wherein: (a) one or more aspartic acid residues is substituted with glutamic acid; (b) one or more isoleucine residues is substituted with leucine; (c) one or more glycine or valine residues is substituted with alanine; (d) one or more arginine residues is substituted with histidine; or (e) one or more tyrosine or phenylalanine residues is substituted with tryptophan.

"Protein modifications or functional fragments" are also encompassed by the term "sumoylation control agent" when it refers to HLS-5 polypeptides. Modifications of HLS-5 polypeptides include homologues, variants and derivatives of the HLS-5 polypeptides, or fragments thereof, which are substantially homologous to primary structural sequence but which include, e.g., *in vivo* or *in vitro* chemical and biochemical modifications or which incorporate unusual amino acids. Such modifications include, for example, acetylation, carboxylation, phosphorylation, glycosylation, ubiquitination, labelling, e.g., with radionucleotides, and various enzymatic modifications, as will be readily appreciated by those well skilled in the art.

A HLS-5 polypeptide "fragment," "portion" or "segment" is a stretch of amino acid residues of at least about five to seven contiguous amino acids, often at least about seven to nine contiguous amino acids, typically at least about nine to 13 contiguous amino acids and, most preferably, at least about 20 to 30 or more contiguous amino acids, wherein said "fragment," "portion" or "segment" has substantially similar function to wild type full length HLS-5 polypeptide.

"Substantially similar function" refers to the function of the polypeptide modification or fragment of HLS-5 with reference to the wild-type HLS-5 polypeptide. The "fragment," "portion" or "segment" of HLS-5 should retain is ability to control the sumoylation proteins as shown by (Boggio et al., 2004, Mol Cell, 16, 549-561). In some embodiments, the "fragment," "portion" or "segment", will comprise one or more domains that have been identified in other proteins as being important with respect to function. For example, the B30.2/SPRY domain and an additional domain in huTRIM5alpha, comprising the amino-terminal RING and B-box components of the TRIM motif, have been shown to be required for N-MLV restriction activity, while the intervening coiled-coil domain is necessary and sufficient for huTRIM5alpha multimerization. Truncated huTRIM5alpha proteins that lack either or both the N-terminal RING/B-Box or the C-terminal B30.2/SPRY domain form heteromultimers with full-length huTRIM5alpha and are dominant inhibitors of its N-MLV restricting activity, suggesting that homomultimerization of intact huTRIM5alpha monomers is necessary for N-MLV restriction. However, localization in large cytoplasmic bodies is not required for inhibition of N-MLV by huTRIM5alpha or for inhibition of HIV-1 by chimeric or rhTRIM5alpha (Yap et al., 2005, Curr Biol, 15, 73-78). Geminin is a cellular protein that associates with Cdt1 and inhibits Mcm2-7 loading during S phase. It prevents multiple cycles of replication per cell cycle and prevents episome replication. Geminin forms a parallel coiled-coil homodimer with atypical residues in the dimer interface. Point mutations that disrupt the dimerization abolish interaction with Cdt1 and inhibition of replication. This interaction is essential for replication inhibition (Saxena et al., 2004, Mol Cell, 15, 245-258). Therefore, it is highly likely that functional fragments, portions or segments of HLS-5 will have one or more of the regions identified above. Moreover, techniques well known in the art for identifying or testing the activity of these regions can be used to test or identify whether the HLS-5 fragment, portion or segment of the present invention is functional.

In addition to the similarity of function, the modified polypeptide may have other useful properties, such as a longer half-life.

In some embodiments, the HLS-5 fragment is an isoform of HLS-5. HLS-5, like other TRIM proteins, are defined by a cluster of three different RBCC or TRIM protein motifs: a RING motif, which is cysteine-rich and binds zinc; one or two so-called B boxes, which also bind zinc; and a coiled-coil domain that is probably involved in the formation of protein complexes. All individual TRIM proteins homo-oligomerization and some might also form alliances with other TRIM proteins (hetero-oligomerization). There are at least 37 TRIM family members in humans (Reymond et al., 2001, Embo J, 20, 2140-2151). Many TRIM family members have alternative splicing, with the best characterised members being TRIM39 (PML) (Duprez et al., 1999, J Cell Sci, 112, 381-393), TRIM18 (MIDI) (Berti et al., 2004, BMC Cell Biol, 5, 9), TRIM32 (LGMD-2H) (Schoser et al., 2005, Ann Neurol, 57, 591-595) and TRIM5 (Xu et al., 2003, Exp Cell Res, 288, 84-93). Each of the various TRIM proteins seems to localize to particular compartments within cells, forming discrete structures to which they entice other proteins, with different isoforms potentially attracting different subsets of proteins and with alternate functions (Reymond *et al.,* 2001, *supra).* Based upon the foregoing, HLS-5 has at least one isoform.

The HLS-5 isoform shown in SEQ ID NO:6 includes an alternate exon in the coding region which results in a frame shift and an early stop codon, compared to HLS-5 shown in SEQ ID NO:4. SEQ ID NO:6 isoform is shorter and has a distinct C-terminus compared to HLS-5 in SEQ ID NO:4.

In certain embodiments, the HLS-5 sumoylation control agents are peptidyl compounds (including peptidomimetics) of HLS-5 which have been modified such that they resist or are more resistant to proteolytic degradation and the like. These peptidyl compounds might include functional groups, such as in place of the scissile peptide bond, which facilitates inhibition of a serine-, cysteine- or aspartate-type protease, as appropriate. For example, the HLS-5 peptidyl compound can be a peptidyl diketone or a peptidyl keto ester, a peptide haloalkylketone, a peptide sulfonyl fluoride, a peptidyl boronate, a peptide epoxide, a peptidyl diazomethanes, a peptidyl phosphonate, isocoumarins, benzoxazin-4-ones, carbamates, isocyantes, isatoic anhydrides or the like. Such functional groups have been provided in other peptide molecules, and general routes for their synthesis are known. See, for example, Angelastro et al., 1990, J. Med Chem. 33:11-13; Bey et al., EPO 363,284; Bey et al., EPO 364,344; Grubb et al., WO 88/10266; Higuchi et al., EPO 393,457; Ewoldt et al., 1992, Molecular Immunology, 29(6):713-721; Hernandez et al., 1992, Journal of Medicinal Chemistry, 35(6): 1121-1129; Vlasak et al., 1989, J. Virology 63(5):2056-2062; Hudig et al., 1991, J. Immunol., 147(4):1360-1368; Odakc et al., 1991, Biochemistry, 30(8):2217-2227; Vijayalakshmi et al., 1991, Biochemistry, 30(8):2175-2183; Kam et al., 1990, Thrombosis & Haemostasis, 64(1):133-137; Powers et al., 1989, J. Cell Biochem., 39(1):33-46; Powers et al., Proteinase Inhibitors, Barrett et al., Eds., Elsevier, pp. 55-152 (1986); Powers et al., 1990, Biochemistry, 29(12):3108-3118; Oweida et al., 1990, Thrombosis Research, 58(2):391-397; Hudig et al., 1989, Molecular Immunology, 26(8):793-798; Orlowski et al., 1989, Archives of Biochemistry & Biophysics, 269(1):125-136; Zunino et al., 1988, Biochimica et Biophysica Acta., 967(3):331-340; Kam et al., 1988, Biochemistry, 27(7):2547-2557; Parkes et al., 1985, Biochem J., 230:509-516; Green et al., 1981, J. Biol. Chem., 256:1923-1928; Angliker et al., 1987, Biochem. J., 241:871-875; Puri et al., 1989, Arch. Biochem. Biophys. 27:346-358; Hanada et al., Proteinase Inhibitors: Medical and Biological Aspects, Katunuma et al., Eds., Springer-Verlag pp. 25-36 (1983); Kajiwara et al., 1987, Biochem. Int., 15:935-944; Rao et al., 1987, Thromb. Res., 47:635-637; Tsujinaka et al., 1988, Biochem. Biophys. Res. Commun. 153:1201-1208). See also US Pat. No. 4,935,493; US Pat. No. 5,462,928; US Pat. No. 5,543,396; US Pat. No. 5,296,604; and US Pat. No. 6,201,132.

In other embodiments, the HLS-5 polypeptide is a non-peptidyl compound, e.g., which can be identified by such drug screening assays as described herein. These non-peptidyl compounds can be, merely to illustrate, synthetic organics, natural products, nucleic acids or carbohydrates.

Also included are such peptidomimetics as olefins, phosphonates, aza-amino acid analogs and the like.

Also deemed as equivalents are any HLS-5-based compounds which can be hydrolytically converted into any of the aforementioned HLS-5 compounds including boronic acid esters and halides, and carbonyl equivalents including acetals, hemiacetals, ketals, and hemiketals, and cyclic dipeptide analogs.

The present invention also encompasses pharmaceutically acceptable salts of the HLS-5 compounds including conventional non-toxic salts or quaternary ammonium salts of the compounds, e.g., from non-toxic organic or inorganic acids. For example, such conventional non-toxic salts include those derived from inorganic acids such as hydrochloride, hydrobromic, sulphuric, sulfonic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

The pharmaceutically acceptable salts of the present invention can be synthesized from the HLS-5 compounds which contain a basic or acid moiety by conventional chemical methods. Generally, the salts are prepared by reacting the free base or acid with stoichiometric amounts or with an excess of the desired salt-forming inorganic or organic acid or base in a suitable solvent.

Contemplated equivalents of the HLS-5 compounds described above include compounds which otherwise correspond thereto, and which have the same general properties thereof (e.g. the ability to control sumoylation), wherein one or more simple variations of substituents are made which do not adversely affect the efficacy of the HLS-5 molecule in use in the contemplated methods. In general, the HLS-5 polypeptides of the present invention may be prepared by the methods described below, or by modifications thereof, using readily available starting materials, reagents and conventional synthesis procedures. In these reactions, it is also possible to make use of variants which are in themselves known, but are not mentioned here.

By the terms "amino acid residue" and "peptide residue" is meant an amino acid or peptide molecule without the -OH of its carboxyl group. In general the abbreviations used herein for designating the amino acids and the protective groups are based on recommendations of the IUPAC-IUB Commission on Biochemical Nomenclature (see Biochemistry (1972) 11:1726-1732). For instance Met, Ile, Leu, Ala and Gly represent "residues" of methionine, isoleucine, leucine, alanine and glycine, respectively. By the residue is meant a radical derived from the corresponding α-amino acid by eliminating the OH portion of the carboxyl group and the H portion of the α-amino group. The term "amino acid side chain" is that part of an amino acid exclusive of the -CH(NH₂)COOH portion, as defined by Kopple, 1966, "Peptides and Amino Acids", WA Benjamin Inc., New York & Amsterdam, pp 2 and 33; examples of such side chains of the common amino acids are -CH₂CH₂SCH₃ (the side chain of methionine), -CH₂(CH₃)-CH₂CH₃ (the side chain of isoleucine), -CH₂CH(CH₃)₂ (the side chain of leucine) or H-(the side chain of glycine).

For the most part, the amino acids used in the application of this invention are those naturally occurring amino acids found in proteins, or the naturally occurring anabolic or catabolic products of such amino acids which contain amino and carboxyl groups.

The term "amino acid residue" further includes analogs, derivatives and congeners of any specific amino acid referred to herein, as well as C-terminal or N-terminal protected amino acid derivatives (eg. modified with an N-terminal or C-terminal protecting group). For example, the present invention contemplates the use of amino acid analogs wherein a side chain is lengthened or shortened while still providing a carboxyl, amino or other reactive precursor functional group for cyclization, as well as amino acid analogs having variant side chains with appropriate, functional groups). For instance, the HLS-5 polypeptide can include an amino acid analog such as, for example, cyanoalanine, canavanine, djenkolic acid, norleucine, 3-phosphoserine, homoserine, dihydroxyphenylalanine, 5-hydroxytryptophan, 1-methylhistidine, 3-methylhistidine, diaminiopimelic acid, ornithine, or diaminobutyric acid. Other naturally occurring amino acid metabolites or precursors having side chains which are suitable herein will be recognized by those skilled in the art and are included in the scope of the present invention.

Also included are the (D) and (L) stereoisomers of such amino acids when the structure of the amino acid admits of stereoisomeric forms. The configuration of the amino acids and amino acid residues herein are designated by the appropriate symbols (D), (L) or (DL), furthermore when the configuration is not designated the amino acid or residue can have the configuration (D), (L) or (DL). It will be noted that the structure of some of the compounds of this invention include asymmetric carbon atoms. It is to be understood accordingly that the isomers arising from such asymmetry are included within the scope of this invention. Such isomers can be obtained in substantially pure form by classical separation techniques and by sterically controlled synthesis. For the purposes of this application, unless expressly noted to the contrary, a named amino acid shall be construed to include both the (D) or (L) stereoisomers.

The phrase "protecting group" as used herein means substituents which protect the reactive functional group from undesirable chemical reactions. Examples of such protecting groups include esters of carboxylic acids and boronic acids, ethers of alcohols and acetals and ketals of aldehydes and ketones. For instance, the phrase "N-terminal protecting group" or "amino-protecting group" as used herein refers to various amino-protecting groups which can be employed to protect the N-terminus of an amino acid or peptide against undesirable reactions during synthetic procedures. Examples of suitable groups include acyl protecting groups such as, to illustrate, formyl, dansyl, acetyl, benzoyl, trifluoroacetyl, succinyl and methoxysuccinyl; aromatic urethane protecting groups as, for example, benzyloxycarbonyl (Cbz); and aliphatic urethane protecting groups such as t-butoxycarbonyl (Boc) or 9-Fluorenylmethoxycarbonyl (FMOC).

Certain polypeptides of the present invention may exist in particular geometric or stereoisomeric forms. The present invention contemplates all such forms, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

The similarity of function (activity) of the modified HLS-5 polypeptide may be substantially the same as the activity of the wild type HLS-5 polypeptide. Alternatively, the similarity of function (activity) of the modified polypeptide may be higher than the activity of the wild-type HLS-5 polypeptide. The function/biological activity of homologues, variant, derivatives or fragments relative to wild type may be determined, for example, by means of biological assays. For example, when administered to HeLa or COS cells, HLS-5 reduces levels of PIAS1, UBC9 and SUMO-1, which results in a reduction of the overall SUMOylation of some protein products and the induction of others. Thus, one *in vivo* SUMOylation assay involves the testing for HLS-5 modulation of protein SUMOylation by the administration of a variant to a HeLa or COS cell, i.e. tissue, etc. and determinating whether the cells have altered levels of SUMOylation of individual protein products by Western analysis. Preferred homologues, variants and fragments are capable of inhibiting SUMOylation by a factor of at least 0.5 relative to full length HLS-5, preferably by a factor of at least 0.9. Another test, based on the interaction of HLS-5 with elements of the SUMOylation machinery is to do *in vitro* SUMO-1 modification assay. Basically, a reaction mixture of 20µl containing 1µg of tagged protein of interest as the substrate as well as 1µg of E1 (GST-Uba2, His₆-Aosl), 2µg of E2 (Ubc9), and 1µg of SUMO-1 in a solution comprising 50mM Tris-HCl (pH 7.5), 50mM NaCl, 10mM ATP, 2mM MgCl₂, and 0.1mM dithiothreitol is incubated for 2h at 30°C (see, for example, Hatakeyama et al., 2001, J Biol Chem, 276, 33111-33120). This assay can be done in the presence or absence of HLS-5. The reaction is terminated by the addition of SDS sample buffer containing 5% β-mercaptoethanol and heating at 88°C for 5min. Samples can then be fractionated by SDS-PAGE on a 12% gel and subjected to immunoblot analysis with mouse monoclonal antibodies to SUMO-1 (2µg/ml, anti-GMP-1), Myc (1µg/ml, 9E10), or GST (1µg/ml), according to the tag used. Immune complexes can be detected with horseradish peroxidase-conjugated rabbit polyclonal antibodies to mouse immunoglobulin. To determine the extent of inhibition of protein substrate, variant or fragment to HLS-5 in an *in vitro* SUMOylation assay. Preferred homologues, variants and fragments are capable of binding to HLS-5 by a factor of at least 0.5 relative to full length HLS-5, preferably by a factor of at least 0.9. Suitable *in vitro* SUMOylation assays are well known to skilled persons, such as 'SUMOylation' assays where one substrate is added to the components of the SUMOylation machinery and the modified or "SUMOylation" products are quantified or observed.

The modified polypeptide may be synthesised using conventional techniques, or is encoded by a modified nucleic acid and produced using conventional techniques. The modified nucleic acid is prepared by conventional techniques. A nucleic acid with a function substantially similar to the wild-type HLS-5 gene function produces the modified protein described above.

Besides substantially full-length polypeptides, the present invention provides for biologically active fragments of the polypeptides. Biologically active fragments are those polypeptide fragments retaining sumoylation controlling activity.

The present invention also provides for fusion polypeptides, comprising HLS-5 polypeptides and fragments. Homologous polypeptides may be fusions between two or more HLS-5 polypeptide sequences or between the sequences of HLS-5 and a related protein. Likewise, heterologous fusions may be constructed which would exhibit a combination of properties or activities of the derivative proteins.

For example, ligand-binding or other domains may be "swapped" between different new fusion polypeptides or fragments. Such homologous or heterologous fusion polypeptides may display, for example, altered strength or specificity of binding. Fusion partners include immunoglobulins, bacterial β galactosidase, trpE, protein A, β-lactamase, alpha amylase, alcohol dehydrogenase and yeast alpha mating factor.

Fusion proteins will typically be made by either recombinant nucleic acid methods, as described below, or may be chemically synthesized.

"Protein purification" refers to various methods for the isolation of the HLS-5 polypeptides from other biological material, such as from cells transformed with recombinant nucleic acids encoding HLS-5, and are well known in the art. For example, such polypeptides may be purified by immuno-affinity chromatography employing, eg., the antibodies provided by the present invention. Various methods of protein purification are well known in the art.

The terms "isolated", "substantially pure", and "substantially homogeneous" are used interchangeably to describe a HLS-5 polypeptide that has been separated from components that accompany it in its natural state. A monomeric protein is substantially purified when at least about 60 to 75% of a sample exhibits a single polypeptide sequence. A substantially purified protein will typically comprise about 60 to 90% W/W of a protein sample, more usually about 95%, and preferably will be over about 99% pure. Protein purity or homogeneity may be indicated by a number of means well known in the art, such as polyacrylamide gel electrophoresis of a protein sample, followed by visualizing a single polypeptide band upon staining the gel. For certain purposes, higher resolution may be provided by using HPLC or other means well known in the art which are utilized for application.

A HLS-5 polypeptide is substantially free of naturally associated components when it is separated from the native contaminants that accompany it in its natural state.

Thus, a HLS-5 polypeptide that is chemically synthesised or synthesised in a cellular system different from the cell from which it naturally originates will be substantially free from its naturally associated components. A protein may also be rendered substantially free of naturally associated components by isolation, using protein purification techniques well known in the art.

A HLS-5 polypeptide produced as an expression product of an isolated and manipulated genetic sequence is an "isolated polypeptide," as used herein, even if expressed in a homologous cell type. Synthetically made forms or molecules expressed by heterologous cells are inherently isolated molecules.

In some embodiments of the present invention the terms "HLS-5 protein" or "HLS-5 polypeptide" refer to a protein or polypeptide encoded by a HLS-5 polynucleotides sequence, or a variant or functional fragment thereof. Also included are HLS-5 polypeptide encoded by DNA that hybridize under high stringency conditions to HLS-5 encoding polynucleotides and closely related polypeptides retrieved by antisera to the HLS-5 protein(s). Accordingly, in some embodiments, the term "sumoylation control agent" comprises an HLS-5 polynucleotide molecule that encodes an HLS-5 polypeptide, allelic variant, or analog, including functional fragments, thereof.

Preferred polynucleotide molecules according to the invention include the polynucleotide sequences set out in SEQ ID NO:1 and SEQ ID NO:3 or functional fragments thereof.

A polynucleotide is said to "encode" a polypeptide if, in its native state or when manipulated by methods well known to those skilled in the art, it can be transcribed and/or translated to produce the corresponding RNA and/or polypeptide, or a fragment thereof. The anti-sense strand is the complement of such a nucleic acid, and the encoding sequence can be deduced therefrom.

An "isolated" or "substantially pure" nucleic acid (e.g., RNA, DNA or a mixed polymer) is one which is substantially separated from other cellular components which naturally accompany a native human sequence or protein, e.g., ribosomes, polymerases, many other human genome sequences and proteins. The term embraces a nucleic acid sequence or protein that has been removed from its naturally occurring environment, and includes recombinant or cloned DNA isolates and chemically synthesized analogs or analogs biologically synthesized by heterologous systems.

"HLS-5 gene sequence," "HLS-5 gene," "HLS-5 nucleic acids" or "HLS-5 polynucleotide" include coding sequences, intervening sequences and regulatory elements controlling transcription and/or translation. The terms are intended to include all allelic variations of the sequence.

These terms refer to a nucleic acid that encodes a HLS-5 polypeptide, fragment, homologue or variant, including, e.g., protein fusions or deletions. The nucleic acids of the present invention will possess a sequence that is either derived from, or substantially similar to a natural HLS-5 encoding gene or one having substantial homology with a natural HLS-5 encoding gene or a portion thereof. The coding sequence for murine HLS-5 polypeptide is shown in SEQ ID NO:1, with the amino acid sequence shown in SEQ ID NO:2. Coding sequence for human HLS-5 polypeptide is shown in SEQ ID NO:3 and SEQ ID NO:7, with the amino acid sequence shown in SEQ ID NO:4 and SEQ ID NO:8.

A nucleic acid or fragment thereof is "substantially homologous" ("or substantially similar") to another if, when optimally aligned (with appropriate nucleotide insertions or deletions) with the other nucleic acid (or its complementary strand), there is nucleotide sequence identity in at least about 60% of the nucleotide bases, usually at least about 70%, more usually at least about 80%, preferably at least about 90%, and more preferably at least about 95-98% of the nucleotide bases.

Alternatively, substantial homology or (identity) exists when a nucleic acid or fragment thereof will hybridise to another nucleic acid (or a complementary strand thereof) under selective hybridisation conditions, to a strand, or to its complement. Selectivity of hybridisation exists when hybridisation that is substantially more selective than total lack of specificity occurs. Typically, selective hybridisation will occur when there is at least about 55% identity over a stretch of at least about 14 nucleotides, preferably at least about 65%, more preferably at least about 75%, and most preferably at least about 90%. The length of homology comparison, as described, may be over longer stretches, and in certain embodiments will often be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides.

Thus, polynucleotides of the invention preferably have at least 75%, more preferably at least 85%, more preferably at least 90% homology to the sequences shown in the sequence listing herein. More preferably there is at least 95%, more preferably at least 98%, homology. Nucleotide homology comparisons may be conducted as described below for polypeptides. A preferred sequence comparison program is the GCG Wisconsin Best fit program described below. The default scoring matrix has a match value of 10 for each identical nucleotide and -9 for each mismatch. The default gap creation penalty is -50 and the default gap extension penalty is -3 for each nucleotide.

In the context of the present invention, a homologous sequence is taken to include a nucleotide sequence which is at least 60, 70, 80 or 90% identical, preferably at least 95 or 98% identical at the amino acid level over at least 20, 50, 100, 200, 300, 500 or 1000 nucleotides with the nucleotides sequences set out in SEQ ID NO:1 or SEQ ID NO:3. In particular, homology should typically be considered with respect to those regions of the sequence that encode contiguous amino acid sequences known to be essential for the function of the protein rather than nonessential neighbouring sequences. Thus, for example, homology comparisons are preferably made over regions corresponding to the Ring finger, B box, coiled coil and/or SPRY domains of the HLS-5 amino acid sequence set out in SEQ ID NO:2, SEQ ID NO:4 or SEQ ID NO:8.

Preferred polypeptides of the invention comprise a contiguous sequence having greater than 50, 60 or 70% homology, more preferably greater than 80, 90, 95 or 97% homology, to one or more of the nucleotides sequences of SEQ ID NO:1 which encode amino acids 111 to 152, 219 to 266 or 368 to 507 of SEQ ID NO:2 or the equivalent nucleotide sequences in SEQ ID NO:3.

Preferred polynucleotides may alternatively or in addition comprise a contiguous sequence having greater than 80,90,95 or 97% homology to the sequence of SEQ ID NO: 1 that encodes amino acids 36 to 75 of SEQ ID NO:2 or the corresponding nucleotide sequences of SEQ ID NO:3. Other preferred polynucleotides comprise a contiguous sequence having greater than 40, 50, 60, or 70% homology, more preferably greater than 80, 90, 95 or 97% homology to the sequence of SEQ ID NO:1 that encodes amino acids 1 to 35, 76 to 110, 153 to 218 and/or 267 to 367 of SEQ ID NO:2 or the corresponding nucleotide sequences of SEQ ID NO:3. Nucleotide sequences are preferably at least 15 nucleotides in length, more preferably at least 20, 30, 40, 50, 100 or 200 nucleotides in length.

Generally, the shorter the length of the polynucleotide, the greater the homology required to obtain selective hybridization. Consequently, where a polynucleotide of the invention consists of less than about 30 nucleotides, it is preferred that the % identity is greater than 75%, preferably greater than 90% or 95% compared with the HLS-5 nucleotide sequences set out in the sequences listed herein.

Conversely, where a polynucleotide of the invention consists of, for example, greater than 50 or 100 nucleotides, the % identity compared with the HLS-5 nucleotide sequences set out in the sequence listings herein may be lower, for example greater than 50%, preferably greater than 60 or 75%.

Nucleic acid hybridisation will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1000mM, typically less than 500mM, and preferably less than 200mM. However, the combination of parameters is much more important than the measure of any single parameter. An example of stringent hybridization conditions is 65°C and 0. 1 x SSC (1 x SSC = 0.15M NaCl, 0.015M sodium citrate pH 7.0).

The "polynucleotide" compositions of this invention include RNA, cDNA, genomic DNA, synthetic forms, and mixed polymers, both sense and antisense strands, and may be chemically or biochemically modified or may contain non-natural or derivatized nucleotide bases, as will be readily appreciated by those skilled in the art. Such modifications include, for example, labels, methylation, substitution of one or more of the naturally occurring nucleotides with an analog, internucleotide modifications such as uncharged linkages (e.g., methyl phosphonates, phosphotriesters, phosphoamidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.). Also included are synthetic molecules that mimic polynucleotides in their ability to bind to a designated sequence via hydrogen bonding and other chemical interactions.

Such molecules are known in the art and include, for example, those in which peptide linkages substitute for phosphate linkages in the backbone of the molecule. The present invention provides recombinant nucleic acids comprising all or part of the HLS-5 region. The recombinant construct may be capable of replicating autonomously in a host cell. Alternatively, the recombinant construct may become integrated into the chromosomal DNA of the host cell. Such a recombinant polynucleotide comprises a polynucleotide of genomic, cDNA, semi-synthetic, or synthetic origin which, by virtue of its origin or manipulation: 1) is not associated with all or a portion of a polynucleotide with which it is associated in nature; and/or 2) is linked to a polynucleotide other than that to which it is linked in nature; or 3) does not occur in nature.

Therefore, recombinant nucleic acids comprising sequences otherwise not naturally occurring are provided by this invention. Although the wild-type sequence may be employed, it will often be altered, e.g., by deletion, substitution or insertion.

A "recombinant nucleic acid" is a nucleic acid that is not naturally occurring, or which is made by the artificial combination of two otherwise separated segments of sequence. This artificial combination is often accomplished by either chemical syntheses means, or by the artificial manipulation of isolated segments of nucleic acids, by genetic engineering techniques. Such is usually done to replace a codon with a redundant codon encoding the same or a conservative amino acid, while typically introducing or removing a sequence recognition site. Alternatively, it is performed to join together nucleic acid segments of desired functions to generate a desired combination of functions. cDNA or genomic libraries of various types may be screened as natural sources of the nucleic acids of the present invention, or such nucleic acids may be provided by amplification of sequences resident in genomic DNA or other natural sources, e.g., by PCR. The choice of cDNA libraries normally corresponds to a tissue source that is abundant in mRNA for the desired proteins. Phage libraries are normally preferred, but other types of libraries may be used. Clones of a library are spread onto plates, transferred to a substrate for screening, denatured and probed for the presence of desired sequences.

The nucleic acid sequences used in this invention will usually comprise at least about five codons (15 nucleotides), more usually at least about 7-15 codons, and most preferably, at least about 35 codons. This number of nucleotides is usually about the minimal length required for a successful HLS-5 fragment that is still capable of controlling sumoylation as described herein.

Techniques for nucleic acid manipulation are described generally, for example, in Sambrook *et al*., 1989, *supra* or Ausubel *et al.,* 1992, Current Protocols in Molecular Biology. Reagents useful in applying such techniques, such as restriction enzymes and the like, are widely known in the art and commercially available from such vendors as New England BioLabs, Boehringer Mannheim, Amersham, Promega Biotec, US Biochemicals, New England Nuclear and a number of other sources. The recombinant nucleic acid sequences used to produce fusion proteins of the present invention may be derived from natural or synthetic sequences. Many natural gene sequences are obtainable from various cDNA or from genomic libraries using appropriate probes. See, GenBank, National Institutes of Health.

As used herein, the term "HLS-5 gene sequence" refers to the double-stranded DNA comprising the gene sequence or region, as well as either of the single-stranded DNAs comprising the gene sequence or region (i.e. either of the coding and non-coding strands).

As used herein, a "portion" of the HLS-5 gene sequence or region is defined as having a minimal size of at least about eight nucleotides, or preferably about 15 nucleotides, or more preferably at least about 25 nucleotides, and may have a minimal size of at least about 40 nucleotides.

A HLS-5 polynucleotide or fragment thereof may be obtained via any known molecular technique. PCR is one such technique that may be used to obtain HLS-5 gene sequences. This technique may amplify, for example, DNA or RNA, including messenger RNA, wherein the DNA or RNA may be single stranded or double stranded. In the event that RNA is to be used as a template, enzymes, and/or conditions optimal for reverse transcribing the template to DNA would be utilized. In addition, a DNA-RNA hybrid that contains one strand of each may be utilized. A mixture of nucleic acids may also be employed, or the nucleic acids produced in a previous amplification reaction described herein, using the same or different primers may be so utilised.

The specific nucleic acid sequence to be amplified, i.e., the HLS-5 gene sequence, may be a fraction of a larger molecule or can be present initially as a discrete molecule, so that the specific sequence constitutes the entire nucleic acid. It is not necessary that the sequence to be amplified is present initially in a pure form; it may be a minor fraction of a complex mixture, such as contained in whole human DNA.

DNA utilized herein may be extracted from a body sample, such as blood, tissue material and the like by a variety of techniques such as that described by Maniatis *et al.* 1982, *supra.* If the extracted sample has not been purified, it may be treated before amplification with an amount of a reagent effective to open the cells, or animal cell membranes of the sample, and to expose and/or separate the strand(s) of the nucleic acid(s). This lysing and nucleic acid denaturing step to expose and separate the strands will allow amplification to occur more readily.

The deoxyribonucleotide triphosphates dATP, dCTP, dGTP and dTTP are added to the synthesis mixture, either separately or together with the primers; in adequate amounts and the resulting solution is heated to about 90°-100°C from about 1 to 10 minutes, preferably from 1 to 4 minutes. After this heating period, the solution is allowed to cool, which is preferable for the primer hybridization. To the cooled mixture is added an appropriate agent for effecting the primer extension reaction (called herein "agent for polymerization"), and the reaction is allowed to occur under conditions known in the art. The agent for polymerization may also be added together with the other reagents if it is heat stable. This synthesis (or amplification) reaction may occur at room temperature up to a temperature above which the agent for polymerization no longer functions.

Thus, for example, if DNA polymerase is used as the agent, the temperature is generally no greater than about 40°C. Most conveniently the reaction occurs at room temperature.

The agent for polymerisation may be any compound or system which will function to accomplish the synthesis of primer extension products, including enzymes.

Suitable enzymes for this purpose include, for example, *E.* coli DNA polymerase I, Klenow fragment of *E. coli* DNA polymerase, polymerase muteins, reverse transcriptase, other enzymes, including heat-stable enzymes (i.e., those enzymes which perform primer extension after being subjected to temperatures sufficiently elevated to cause denaturation), such as *Taq* polymerase. A suitable enzyme will facilitate combination of the nucleotides in the proper manner to form the primer extension products that are complementary to each HLS-5 gene sequence nucleic acid strand. Generally, the synthesis will be initiated at the 3' end of each primer and proceed in the 5' direction along the template strand, until synthesis terminates, producing molecules of different lengths.

The newly synthesised HLS-5 strand and its complementary nucleic acid strand will form a double-stranded molecule under hybridizing conditions described above and this hybrid is used in subsequent steps of the process. In the next step, the newly synthesized HLS-5 double-stranded molecule is subjected to denaturing conditions using any of the procedures described above to provide single-stranded molecules.

The steps of denaturing, annealing, and extension product synthesis can be repeated as often as needed to amplify the target polymorphic gene sequence nucleic acid sequence to the extent necessary for detection. The amount of the specific nucleic acid sequence produced will accumulate in an exponential fashion. Amplification is described, for example, in "PCR. A Practical Approach", ILR Press, Eds. McPherson et al., 1992.

Sequences amplified by the methods of the invention can be further evaluated, detected, cloned, sequenced, and the like, either in solution or after binding to a solid support, by any method usually applied to the detection of a specific DNA sequence such as PCR, oligomer restriction (Saiki et al., 1985, Bio/Technology, 3: 1008-1012), allele-specific oligonucleotide (ASO) probe analysis (Conner et al., 1983, Proc. Natl. Acad. Sci. USA., 80: 278), oligonucleotide ligation assays(OLAs) (Landgren et al., 1988, Science, 241: 1007) and the like. Molecular techniques for DNA analysis have been reviewed (Landgren et al., 1988, Science, 242: 229-237).

Methods of obtaining HLS-5 polynucleotides of the present invention include PCR, as described herein and as commonly used by those of ordinary skill in the art. Alternative methods of amplification have been described and can also be employed as long as the HLS-5 gene sequence amplified by PCR using primers of the invention is similarly amplified by the alternative means. Such alternative amplification systems include but are not limited to self-sustained sequence replication, which begins with a short sequence of RNA of interest and a T7 promoter. Reverse transcriptase copies the RNA into cDNA and degrades the RNA, followed by reverse transcriptase polymerizing a second strand of DNA. Another nucleic acid amplification technique is nucleic acid sequence-based amplification (NASBA) which uses reverse transcription and T7 RNA polymerase and incorporates two primers to target its cycling scheme. NASBA can begin with either DNA or RNA and finish with either, and amplifies to 108 copies within 60 to 90 minutes.

Alternatively, HLS-5 polynucleotides can be amplified by ligation activated transcription (LAT). LAT works from a single-stranded template with a single primer that is partially single-stranded and partially double-stranded. Amplification is initiated by ligating a cDNA to the promoter oligonucleotide and within a few hours, amplification is 108 to 109 fold. The QB replicase system can be utilized by attaching an RNA sequence called MDV-1 to RNA complementary to a DNA sequence of interest. Upon mixing with a sample, the hybrid RNA finds its complement among the specimen's mRNA and binds, activating the replicase to copy the tag-along sequence of interest. Another nucleic acid amplification technique, ligase chain reaction (LCR), works by using two differently labelled halves of a sequence of interest that are covalently bonded by ligase in the presence of the contiguous sequence in a sample, forming a new target. The repair chain reaction (RCR) nucleic acid amplification technique uses two complementary and target-specific oligonucleotide probe pairs, thermostable polymerase and ligase, and DNA nucleotides to geometrically amplify targeted sequences. A 2-base gap separates the oligonucleotide probe pairs, and the RCR fills and joins the gap, mimicking normal DNA repair. Nucleic acid amplification by strand displacement activation (SDA) utilizes a short primer containing a recognition site for *Hinc* II with short overhang on the 5'end that binds to target DNA. A DNA polymerase fills in the part of the primer opposite the overhang with sulphur-containing adenine analogs. *Hinc* II is added but only cuts the unmodified DNA strand. A DNA polymerase that lacks 5'exonuclease activity enters at the site of the nick and begins to polymerize, displacing the initial primer strand downstream and building a new one which serves as more primer. SDA produces greater than 107-fold amplification in 2 hours at 37°C. Unlike PCR and LCR, SDA does not require instrumented temperature cycling. Another amplification system useful in the method of the invention is the QB Replicase System. Although PCR is the preferred method of amplification used in the invention, these other methods can also be used to amplify the HLS-5 gene sequence as described in the method of the invention.

Large amounts of the HLS-5 polynucleotides of the present invention may also be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually the polynucleotide constructs will be suitable for replication in a unicellular host, such as yeast or bacteria, but may also be intended for introduction to (with and without integration within the genome) cultured mammalian or plant or other eukaryotic cell lines.

A double-stranded fragment may be obtained from the single-stranded product of chemical synthesis either by synthesizing the complementary strand and annealing the strands together under appropriate conditions or by adding the complementary strand using DNA polymerase with an appropriate primer sequence.

HLS-5 polynucleotides of the invention may be incorporated into a recombinant replicable vector for introduction into a prokaryotic or eukaryotic host. Such vectors may typically comprise a replication system recognized by the host, including the intended polynucleotide fragment encoding the desired polypeptide, and will preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Secretion signals may also be included where appropriate, whether from a native HLS-5 protein or from other receptors or from secreted polypeptides of the same or related species, which allow the protein to cross and/or lodge in cell membranes, and thus attain its functional topology, or be secreted from the cell. Such vectors may be prepared by means of standard recombinant techniques well known in the art and discussed, for example, in Sambrook *et al*., 1989 *supra* or Ausubel *et al.* 1992 *supra.*

An appropriate promoter and other necessary vector sequences will be selected so as to be functional in the host, and may include, when appropriate, those naturally associated with HLS-5 genes. Examples of workable combinations of cell lines and expression vectors are described in Sambrook *et al*., 1989 *supra* or Ausubel *et al*., 1992. Many useful vectors are known in the art and may be obtained from such vendors as Stratagene, New England Biolabs, Promega, Biotech, and others. Promoters such as the trp, lac and phage promoters, tRNA promoters and glycolytic enzyme promoters may be used in prokaryotic hosts.

Useful yeast promoters include promoter regions for metallothionein, phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in Hitzeman et al., 1983, Science, 219, pages 620-625.

Appropriate non-native mammalian promoters might include the early and late promoters from SV40 or promoters derived from murine Moloney leukemia virus, mouse tumour virus, avian sarcoma viruses, adenovirus 11, bovine papilloma virus or polyoma. In addition, the construct may be joined to an amplifiable gene (e.g., DHFR) so that multiple copies of the gene may be made.

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells that express the inserts. Typical selection genes encode proteins that a) confer resistance to antibiotics or other toxic substances, e.g. ampicillin, neomycin, methotrexate, etc., b) complement auxotrophic deficiencies, or c) supply critical nutrients not available from complex media, e.g., the gene encoding D-alanine racemase for *Bacilli*. The choice of the proper selectable marker will depend on the host cell, and appropriate markers for different hosts are well known in the art.

The vectors containing the nucleic acids of interest can be transcribed *in vitro*, and the resulting RNA introduced into the host cell by well-known methods, e.g., by injection, or the vectors can be introduced directly into host cells by methods well known in the art, which vary depending on the type of cellular host, including: electroporation; transfection employing calcium chloride, rubidium chloride, calcium phosphate, DEAE-dextran, or other substances; microprojectile bombardment; lipofection; infection (where the vector is an infectious agent, such as a retroviral genome); and other methods. The introduction of the polynucleotides into the host cell by any method known in the art, including, *inter alia,* those described above, will be referred to herein as "transformation". The cells into which the polynucleotides have been introduced include the progeny of such cells.

Thus the present invention provides host cells transformed or transfected with a nucleic acid molecule of the invention. Preferred host cells include bacteria, yeast, mammalian cells, plant cells, insect cells, and human cells.

Illustratively, such host cells are selected from the group consisting of *E. coli, Pseudomonas, Bacillus, Streptomyces,* yeast, CHO, R1.1, B-W, L-M, COS 1, COS 7, BSC1, BSC40,BMT10, and Sf9 cells.

Large quantities of the HLS-5 polypeptides of the present invention may be prepared by expressing the HLS-5 polynucleotides or portions thereof in vectors or other expression vehicles in compatible prokaryotic or eukaryotic host cells. The most commonly used prokaryotic hosts are strains of *Escherichia coli,* although other prokaryotes, such as *Bacillus subtilis* or *Pseudomonas* may also be used.

Also provided are mammalian cells containing an HLS-5 polypeptide encoding DNA sequence and modified *in vitro* to permit higher expression of HLS-5 polypeptide by means of a homologous recombinational event consisting of inserting an expression regulatory sequence in functional proximity to the HLS-5 polypeptide encoding sequence. The expression regulatory sequence can be an HLS-5 polypeptide expression regulatory sequence, or not, and can replace a mutant HLS-5 polypeptide regulatory sequence in the cell.

Thus, the present invention also provides methods for preparing an HLS-5 polypeptide comprising: (a) culturing a cell as described above under conditions that provide for expression of the HLS-5 polypeptide; and (b) recovering the expressed HLS-5 polypeptide. This procedure can also be accompanied by the steps of: (c) chromatographing the polypeptide using any suitable means known in the art; and (d) purifying the polypeptide by, for example, gel filtration.

Mammalian or other eukaryotic host cells, such as those of yeast, filamentous fungi, plant, insect, or amphibian or avian species, may also be useful for production of the proteins of the present invention. Propagation of mammalian cells in culture is *per se* well known. Examples of commonly used mammalian host cell lines are VERO and HeLa cells, Chinese hamster ovary (CHO) cells, and W138, BHK, and COS cell lines, although it will be appreciated by the skilled practitioner that other cell lines may be appropriate, e.g., to provide higher expression, desirable glycosylation patterns, or other features.

Clones are selected by using markers depending on the mode of the vector construction. The marker may be on the same or a different DNA molecule, preferably the same DNA molecule. In prokaryotic hosts, the transformant may be selected, e.g., by resistance to ampicillin, tetracycline or other antibiotics.

Production of a particular product based on temperature sensitivity may also serve as an appropriate marker.

Prokaryotic or eukaryotic cells transformed with the polynucleotides of the present invention will be useful not only for the production of the nucleic acids and polypeptides of the present invention.

In some embodiments the "sumoylation control agent" is a compound or composition capable of modifying the endogenous level(s) of HLS-5 and/or HLS-5 activity. The term "modifying" as used herein with reference to the endogenous levels of HLS-5 refers to the ability of the compound or composition to increase or decrease the endogenous levels of HLS-5 and/or HLS-5 activity as compared to the wild-type and/or normal levels. Compounds and/or compositions capable of modifying the endogenous levels of HLS-5 and/or HLS-5 activity can be initially identified using *in vitro* cell based assays. For example, a system such as Chroma-Luc™, Luc™ or GFP™ reporter genes can be provided in multiple different cloning vector formats. The Basic vector versions are general-purpose reporter vectors based on the design, for example of the pGL3-Basic Vector, which lacks eukaryotic promoter and enhancer sequences, allowing cloning of putative regulatory sequences, such as the HLS-5 promoter at the 5' end of the reporter gene. Expression of luciferase, or any reporter gene, activity in cells transfected with this "pGL3-Promoter Vector" depends on elements or compounds being able to induce directly or indirectly the expression through the cloned promoter of interest, such as the HLS-5 promoter. In addition to the basic vector configuration, other systems such as the Chroma-Luc™ genes are available in a vector configuration containing an SV40 promoter and SV40 enhancer, similar to the pGL3-Control Vector. The presence of the SV40 promoter and enhancer sequences result in strong expression of *luc*+ in many types of mammalian cells. Thus this technology and any other vector modification is suitable for rapid quantitation in multiwell plates and in high-throughput applications to assay for compounds which are potentially capable of modifying the HLS-5 protein expression by measuring the reporter gene downstream of the HLS-5 promoter. These identified compounds can then be tested in cells with the endogenous HLS-5 promoter and protein expression assayed by such methods as Western Blots. In general, any luminometer capable of measuring filtered luminescence should be able to perform dual-colour assays and any scientist skilled in the art can reproduce these assays.

A modifier of HLS-5 may be detected by contacting a sumoylation-conjugating system with HLS-5 and measuring the level of sumoylation of the substrate polypeptide in the presence of HLS-5. As used herein the term "contacting" includes direct and indirect contacting of the sumoylation-conjugating agent and HLS-5. The contacting may be by any means known in the art, for example, by adding the sumoylation-conjugating agent to the HLS-5 or vice versa. The term "measuring" as used herein includes any method of measuring the level of sumoylation, including the method of Collavin et al, 2004, *supra*), and may detect an upregulation or down regulation (ie modulated level) of endogenous HLS-5. "Endogenous HLS-5" refers to the HLS-5 produced by the body, ie is not from an external source.

The modulated level of HLS-5 may be statistically significant ie., greater than what might be expected to happen by chance alone. Significance is typically defined by an appropriately small p value, such as p<0.05. A statistically significant finding may not necessarily be clinically significant, ie, it does not have a measurable impact on patients' symptoms.

Once the sumoylation control agents eg HLS-5 polypeptide, HLS-5 polynucleotide in appropriate vector and/or compound/composition capable of modifying the endogenous levels of HLS-5 and/or HLS-5 activity, have been obtained they are then administered to a subject in order to regulate sumoylation. In some embodiments, the subject is suffering from a condition, which is affected by, controlled by, or exacerbated by sumoylation and therefore, the step of administration assists in the treatment of the condition.

Generally, the terms "treating," "treatment" and the like are used herein to mean affecting a subject e.g. human individual or animal, its tissue or cells to obtain a desired pharmacological and/or physiological effect. The effect may be prophylactic in terms of completely or partially preventing the condition or sign or symptom thereof, and/or may be therapeutic in terms of a partial or complete cure of the condition. "Treating" as used herein covers any treatment of, or prevention of a condition associated with or exacerbated by sumoylation in a vertebrate, a mammal, particularly a human, and includes: (a) preventing the condition from occurring in a subject that may be predisposed to the condition, but has not yet been diagnosed as having it; (b) inhibiting the condition, i.e., arresting its development; or (c) relieving or ameliorating the condition, i.e., cause regression of the symptoms.

The term "subject" as used herein refers to an animal subject in which the control of sumoylation activity is desirable. The subject may be a human, or may be a domestic, companion or zoo animal. While it is particularly contemplated that the sumoylation control agents of the invention are suitable for use in medical treatment of humans, they are also applicable to veterinary treatment, including treatment of companion animals such as dogs and cats, and domestic animals such as horses, cattle and sheep, or zoo animals such as non-human primates, felids, canids, bovids, and ungulates.

The sumoylation control agent(s) can be administered in various forms, depending on the condition to be treated and the age, condition and body weight of the subject, as is well known in the art. For example, where the sumoylation control agent(s) are to be administered orally, they may be formulated as tablets, capsules, granules, powders or syrups; or for parenteral administration, they may be formulated as injections (intravenous, intramuscular or subcutaneous), drop infusion preparations or suppositories. For application by the ophthalmic mucous membrane route, they may be formulated as eye drops or eye ointments. These formulations can be prepared by conventional means, and, if desired, the active ingredient may be mixed with any conventional additive, such as an excipient, a binder, a disintegrating agent, a lubricant, a corrigent, a solubilizing agent, a suspension aid, an emulsifying agent or a coating agent. Although the dosage will vary depending on the symptoms, age and body weight of the subject, the nature and severity of the condition to be treated or prevented, the route of administration and the form of the sumoylation control agent, in general, a daily dosage of from 0.01 to 2000 mg of the sumoylation control agents is recommended for an adult human subject, and this may be administered in a single dose or in divided doses. An effective time for administering the sumoylation control agent needs to be identified. This can be accomplished by routine experiments. For example, in animals, the control of sumoylation activity by the sumoylation control agent can be assessed by administering the sumoylation control agent at a particular time of day and measuring the effect of the administration (if any) by measuring one or more indices associated with sumoylation, and comparing the post-treatment values of these indices to the values of the same indices prior to treatment.

The precise time of administration and/or amount of sumoylation control agent that will yield the most effective results in terms of efficacy of treatment in a given subject will depend upon the activity, pharmacokinetics, and bioavailability of a particular sumoylation control agent, physiological condition of the subject (including age, sex, disease type and stage, general physical condition, responsiveness to a given dosage and type of medication), route of administration, etc. However, the above guidelines can be used as the basis for fine-tuning the treatment, eg., determining the optimum time and/or amount of administration, which will require no more than routine experimentation comprising monitoring the subject and adjusting the dosage and/or timing.

The phrases "pharmaceutically-effective amount" and "therapeutically-effective amount" as used herein means that amount of a sumoylation control agent, which is effective for producing some desired pharmaceutical and/or therapeutic effect, for example, the inhibition of sumoylation of a protein at a reasonable benefit/risk ratio applicable to any medical treatment.

The phrase "pharmaceutically acceptable" is employed herein to refer to those sumoylation control agents, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition or vehicle, such as a liquid or solid filler, diluent, excipient, solvent or encapsulating material, involved in carrying or transporting the sumoylation control agents from one organ, or portion of the body, to another organ, or portion of the body. Each carrier must be "acceptable" in the sense of being compatible with the other ingredients of the formulation and not injurious to the subject. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Wetting agents, emulsifiers and lubricants, such as sodium lauryl sulfate and magnesium stearate, as well as colouring agents, release agents, coating agents, sweetening, flavouring and perfuming agents, preservatives and antioxidants can also be present in formulations of the invention.

Examples of pharmaceutically-acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin; propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations useful in the methods of the present invention include those suitable for oral, nasal, topical (including buccal and sublingual), rectal, vaginal, aerosol and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated and the particular mode of administration. The amount of active ingredient which can be combined with a carrier material to produce a single dosage form will generally be that amount of the compound which produces a therapeutic effect. Generally, out of one hundred per cent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a sumoylation control agent(s) with the carrier and, optionally, one or more accessory ingredients. In general, the formulations are prepared by uniformly and intimately bringing into association a sumoylation control agent with liquid carriers, or finely divided solid carriers, or both, and then, if necessary, shaping the product.

Formulations suitable for oral administration may be in the form of capsules, cachets, pills, tablets, lozenges (using a flavoured basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatine and glycerine, or sucrose and acacia) and/or as mouth washes and the like, each containing a predetermined amount of a sumoylation control agent(s) as an active ingredient. A compound may also be administered as a bolus, electuary or paste.

In solid dosage forms for oral administration (capsules, tablets, pills, powders, granules and the like), the active ingredient is mixed with one or more pharmaceutically-acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) colouring agents. In the case of capsules, tablets and pills, the pharmaceutical compositions may also comprise buffering agents. Solid compositions of a similar type, may also be employed as fillers in soft and hard-filled gelatine capsules using such excipients as lactose or milk sugars, as well as high molecular weight polyethylene glycols and the like.

A tablet may be made by compression or moulding, optionally with one or more accessory ingredients. Compressed tablets may be prepared using binder (for example, gelatine or hydroxypropylmethyl cellulose), lubricant, inert diluent, preservative, disintegrant (for example, sodium starch glycolate or cross-linked sodium carboxymethyl cellulose), surface-active or dispersing agent. Moulded tablets may be made by moulding in a suitable machine a mixture of the powdered peptide or peptidomimetic moistened with an inert liquid diluent.

Tablets, and other solid dosage forms, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration include pharmaceutically acceptable emulsions, microemulsions, solutions, suspensions, syrups and elixirs. In addition to the active ingredient, the liquid dosage forms may contain inert diluents commonly used in the art, such as, for example, water or other solvents, solubilizing agents and emulsifiers, such as ethyl alcohol, isodropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavouring, colouring, perfuming and preservative agents

Suspensions, in addition to the active sumoylation control agent(s) may contain suspending agents as, for example, ethoxylated isostearyl alcohols, polyoxyethylene sorbitol and sorbitan esters, microcrystalline cellulose, aluminium metahydroxide, bentonite, agar-agar and tragacanth, and mixtures thereof.

Dosage forms for the topical or transdermal administration of a sumoylation control agent(s) include powders, sprays, ointments, pastes, creams, lotions, gels, solutions, patches and inhalants. The active component may be mixed under sterile conditions with a pharmaceutically-acceptable carrier, and with any preservatives, buffers, or propellants which may be required.

The ointments, pastes, creams and gels may contain, in addition to sumoylation control agent(s), excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a sumoylation control agent(s), excipients such as lactose, talc, silicic acid, aluminium hydroxide, calcium silicates and polyamide powder, or mixtures of these substances. Sprays can additionally contain customary propellants, such as chlorofluorohydrocarbons and volatile unsubstituted hydrocarbons, such as butane and propane.

The sumoylation control agent(s) can be alternatively administered by aerosol. This is accomplished by preparing an aqueous aerosol, liposomal preparation or solid particles containing the compound. A non-aqueous (e.g., fluorocarbon propellant) suspension could be used. Sonic nebulizers are preferred because they minimize exposing the agent to shear, which can result in degradation of the compound.

Ordinarily, an aqueous aerosol is made by formulating an aqueous solution or suspension of the agent together with conventional pharmaceutically acceptable carriers and stabilizers. The carriers and stabilizes vary with the requirements of the particular compound, but typically include non-ionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Transdermal patches have the added advantage of providing controlled delivery of a sumoylation control agent(s) to the body. Such dosage forms can be made by dissolving or dispersing the agent in the proper medium. Absorption enhancers can also be used to increase the flux of the peptidomimetic across the skin. The rate of such flux can be controlled by either providing a rate controlling membrane or dispersing the peptidomimetic in a polymer matrix or gel.

Ophthalmic formulations, eye ointments, powders, solutions and the like, are also contemplated as being within the scope of this invention.

Pharmaceutical compositions of this invention suitable for parenteral administration comprise one or more sumoylation control agent(s) in combination with one or more pharmaceutically-acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various anti-bacterial and anti-fungal agents, for example, paraben, chlorobutanol, phenol sorbic-acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminium monostearate and gelatine.

In some cases, in order to prolong the effect of a sumoylation control agent, it is desirable to slow the absorption of the agent from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered agent form is accomplished by dissolving or suspending the agent in an oil vehicle.

Injectable depot forms are made by forming microencapsule matrices of sumoylation control agent(s) in biodegradable polymers such as polylactide-polyglycolide. Depending on the ratio of agent to polymer, and the nature of the particular polymer employed, the rate of agent release can be controlled. Examples of other biodegradable polymers include poly(orthoesters) and poly(anhydrides). Depot injectable formulations are also prepared by entrapping the agent in liposomes or microemulsions which are compatible with body tissue.

The phrases "parenteral administration" and "administered parenterally" as used herein mean modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion administration.

The phrases "systemic administration," "administered systemically," "peripheral administration" and "administered peripherally" as used herein mean the administration of a sumoylation control agent other than directly into the central nervous system, such that it enters the subject's system and, thus, is subject to metabolism and other like processes, for example, subcutaneous administration.

Another aspect of the invention provides a conjoint therapy wherein one or more other therapeutic agents are administered with the sumoylation control agent. Such conjoint treatment may be achieved by way of the simultaneous, sequential or separate dosing of the individual components of the treatment.

In some embodiments, a sumoylation control agent is conjointly administered with anti-cancer agents or other therapeutic agents known to be useful in the treatment of the condition being treated. For example, gene therapy using HLS-5 expression vector together with an anti-cancer agent.

In another illustrative embodiment, the subject control agents can be conjointly administered with a sumoylation agonist or antagonist.

As described *supra,* in some embodiments the HLS-5 polynucleotides and vectors of the invention for *in vivo* delivery and expression. This approach has also been called "gene therapy" and as such is well known in the art. Gene therapy protocols may involve administering an therapeutically-effective amount of a HLS-5 polynucleotide vector capable of directing expression of the HLS-5 polypeptide to a subject either before, substantially contemporaneously, with, or after influenza virus infection. Another approach that may be used alternatively or in combination with the foregoing is to isolate a population of cells, e.g., stem cells or immune system cells from a subject, optionally expand the cells in tissue culture, and administer a HLS-5 polynucleotide vector capable of directing expression of HLS-5 to the cells *in vitro.* The cells may then be returned to the subject. Optionally, cells expressing the HLS-5 polynucleotides can be selected *in vitro* prior to introducing them into the subject. In some embodiments of the invention a population of cells, which may be cells from a cell line or from an individual who is not the subject, can be used. Methods of isolating stem cells, immune system cells, etc., from a subject and returning them to the subject are well known in the art. Such methods are used, eg., for bone marrow transplant, peripheral blood stem cell transplant, etc., in patients undergoing chemotherapy.

In yet another approach, oral gene therapy may be used. For example, US. Pat. No. 6,248,720 describes methods and compositions whereby genes under the control of promoters are protectively contained in microparticles and delivered to cells in operative form, thereby achieving non-invasive gene delivery. Following oral administration of the microparticles, the genes are taken up into the epithelial cells, including absorptive intestinal epithelial cells, taken up into gut associated lymphoid tissue, and even transported to cells remote from the mucosal epithelium. As described therein, the microparticles can deliver the genes to sites remote from the mucosal epithelium, i.e. can cross the epithelial barrier and enter into general circulation, thereby transfecting cells at other locations.

The term "condition" refers to any disease or disorder in which sumoylation plays a part and requires controlling. At present there are a number of conditions known to be affected by sumoylation including, but not limited to, Parkinson's disease, diabetes, Huntington's disease, familial neuronal intranuclear inclusion disease, Alzheimer's disease, neuronal intranuclear inclusion disease, cancer, polyglutamine disease, and HIV infection.

In Parkinson's disease, parkin is a ubiquitin ligase and gives special meaning to the molecular mechanism of neurodegeneration in general. In Parkinson's disease, Lewy bodies are immunoreactive for ubiquitin. Accumulation of abnormal proteins has also been seen in other neurodegenerative disorders. It has been postulated that disturbance of protein degradation by the ubiquitin-proteasome system might have a critical role in neurodegeneration. Although alpha-synuclein mutations are infrequent, alpha-synuclein accumulates in Lewy bodies, and alpha-synuclein fibrils impair the 26S proteasome function. UCH-L1 is also an abundant deubiquitylating enzyme, and its mutation is linked to PARK5. Furthermore, DJ-1 interacts with SUMO-1, which can counteract ubiquitin and stabilise proteins against degradation by the 26S proteasome (Hattori & Mizuno, 2004, Lancet, 364, 722-724).

In some cases of diabetes, a single nucleotide polymorphism in SUMO-4 has been detected, substituting a highly conserved methionine with a valine residue (M55V). In HepG2 (liver carcinoma) cells transiently transfected with SUMO-4 expression vectors, Met-55 was associated with the elevated levels of activated heat shock factor transcription factors as compared with Val-55, whereas the levels of NF-kappaB were suppressed to an identical degree. The SUMO-4M (Met) variant is associated with type I diabetes mellitus susceptibility in families, suggesting that it may be involved in the pathogenesis of type I diabetes (Bohren et al., 2004, J Biol Chem, 279, 27233-27238). Huntington's disease (HD) is characterized by the accumulation of a pathogenic protein, Huntingtin (Htt) that contains an abnormal polyglutamine expansion. A pathogenic fragment of Htt (Httexlp) is known to be modified either by SUMO-1 or by ubiquitin on identical lysine residues. In cultured cells, SUMOylation stabilizes Httex1p, reduces its ability to form aggregates, and promotes its capacity to repress transcription. In a Drosophila model of HD, SUMOylation of Httex1p exacerbates neurodegeneration, whereas ubiquitination of Httex1p abrogates neurodegeneration. Lysine mutations that prevent both SUMOylation and ubiquitination of Httex1p reduce HD pathology, indicating that the contribution of SUMOylation to HD pathology extends beyond preventing Htt ubiquitination and degradation (Steffan *et al.,* 2004, *supra*).

Neuronal intranuclear inclusion disease (NIID) is a rare neurodegenerative disorder characterized by progressive ataxia and neuronal nuclear inclusions (NIs), similar to the inclusions found in expanded CAG repeat diseases. NIID may be familial or sporadic. The cause of familial NIID is poorly understood, as no CAG expansion has been detected. NIs in all three cases were intensely immunopositive for SUMO-1, Glucocorticoid Receptor and Ubiquitin. Electron microscopy demonstrated that SUMO-1 was located on the 10-nm fibrils of NIs. In cultured PC12 cells, it has been shown that inhibition of proteasome function by specific inhibitors resulted in the appearance of SUMO-1-immunopositive nuclear inclusions. This suggests that recruitment of SUMO-1 modified proteins into insoluble nuclear inclusions and proteasomal dysfunction may be involved in the pathogenesis of NIs in familial NIID cases (Pountney et *al.,* 2003, *supra).* Amyloid beta peptide (Abeta) generated from amyloid precursor protein (APP) is central to Alzheimer's disease (AD). Signaling pathways affecting APP amyloidogenesis play critical roles in AD pathogenesis and can be exploited for therapeutic intervention. It has been demonstrated that sumoylation regulates Abeta generation. Increased protein sumoylation resulting from over-expression of SUMO-3 dramatically reduces Abeta production. In addition, SUMO-3 immunoreactivity is predominantly detected in neurons in brains from AD, Down's syndrome, and non-demented humans. Therefore, polysumoylation reduces whereas monosumoylation or undersumoylation enhances Abeta generation. These findings provide a regulatory mechanism in APP amyloidogenesis and suggest that components in the sumoylation pathway may be critical in AD onset or progression (Lu et *al.,* 2003, *supra).*

Juvenile NIID is a spinocerebellar brainstem ataxic disease possibly related to an abnormality in SUMOylation (McFadden et al., 2005, J Neuropathol Exp Neurol, 64, 545-552). Patients of DRPLA, SCA1, MJD, and Huntington's disease are found to have neurons in affected regions of the brain which react strongly to SUMO-1. Western blot with a transgenic mouse expressing mutant ataxin-1 showed the increase of SUMOylated proteins in the cerebellar cortex, named ESCA1 and ESCA2. Activation of SUMO-1 system in polyglutamine diseases and predicted its involvement in the pathology (Ueda *et al.,* 2002, *supra).* The p6 domain of the human immunodeficiency virus type 1 (HIV-1) Gag polyprotein mediates virion budding from infected cells via protein-protein contacts with the class E vacuolar protein sorting factors, Tsg101 and AIP1/ALIX. Interaction with Tsg101 is strengthened by covalent attachment of monovalent ubiquitin to HIV-1 p6. HIV-1 p6 was found to interact with SUMO-1 as well as the E2 SUMO-1 transfer enzyme, Ubc9. Interaction with p6 was also detected with Daxx, a cellular protein to which SUMO-1 is sometimes covalently attached. SUMO-1 was incorporated into HIV-1 virions where it was protected within the virion membrane from digestion by exogenous protease. Of the two lysine residues in p6, lysine 27 uniquely served as a site of covalent SUMO-1 attachment. As previously reported, though, HIV-1 bearing the p6-K27R mutation replicated just like the wild type. Overproduction of SUMO-1 in HIV-1 producer cells had no apparent effect on virion release or on virion protein or RNA content. Infectivity of the resulting virions, though, was decreased, with the defect occurring after membrane fusion, at the time of viral cDNA synthesis, suggesting that covalent attachment of SUMO-1 to p6 is detrimental to HIV-1 replication (Gurer et al., 2005, J Virol, 79, 910-917).

The present invention also provides assays that are suitable for identifying substances that bind to HLS-5 polypeptides (reference to which includes homologues, variants, derivatives and fragments as described above). In addition, assays are provided that are suitable for identifying substances that interfere with HLS-5 binding to cellular components involved in sumoylation, for example proteins identified in yeast two-hybrid screens as interacting with HLS-5. Such assays are typically *in vitro*. Assays are also provided that test the effects of candidate substances identified in preliminary *in vitro* assays on intact cells in whole cell assays.

A substance that alters sumoylation as a result of an interaction with HLS-5 polypeptides may do so in several ways. It may directly disrupt the binding of HLS-5 to a cellular component of the cell cycle machinery by, for example, binding to HLS-5 and masking or altering the site of interaction with the other component. Candidate substances of this type may conveniently be preliminarily screened by *in vitro* binding assays as, for example, described below and then tested, for example in a whole cell assay as described below.

As mentioned previously, when administered to cells or when its expression levels are high, HLS-5 reduces levels of PIAS1, UBC9 and SUMO-1, resulting in a reduction of the overall SUMOylation of some protein targets and the induction of others. Thus an *in vivo* SUMOylation assay is one test for HLS-5 modulation of protein SUMOylation and can be done by administration to a variant of HeLa or COS cell, for example, etc. and determine whether cells have altered levels of SUMOylation of individual protein products by Western analysis. Another test, based on the interaction of HLS-5 with elements of the SUMOylation machinery is an *in vitro* SUMO-1 modification assay as described *supra.*

By "comprising" is meant including, but not limited to, whatever follows the word comprising". Thus, use of the term "comprising" indicates that the listed elements are required or mandatory, but that other elements are optional and may or may not be present. By "consisting of" is meant including, and limited to, whatever follows the phrase "consisting of". Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The following examples, which describe exemplary techniques and experimental results, are provided for the purpose of illustrating the invention, and should not be construed as limiting.

### EXAMPLE 1 HLS-5 BINDS COMPONENTS OF THE SUMOYLATION MACHINERY

A yeast two-hybrid screen for HLS-5 interacting partners identified two proteins involved in the sumoylation pathway viz Ubc9 and PIAS1. Ubc9 has been shown to act as an E2 enzyme in the SUMO-1 conjugation pathway (Desterro et al., 1997, FEBS Lett, 417, 297-300, Johnson *et al*., 1997, *supra),* while PIAS1 has been demonstrated to act as an E3 ligase in that reaction sequence for multiple proteins such as p53, Sp3 and the androgen receptor (Kahyo et al., 2001, Mol Cell, 8, 713-718; Nishida & Yasuda, 2002, *supra*; Sapetschnig et al., 2002, Embo J, 21, 5206-5215) .

The interaction between HLS-5 and Ubc9 was characterized further by yeast 2 hybrid assays to define important binding domains. These data are summarised in Figure 1a and indicate several regions of HLS-5 were able to associate with Ubc9. To confirm that HLS-5 and Ubc9 were genuine binding partners, the interaction was investigated in mammalian cells using bioluminescence resonance energy transfer (BRET), which is able to detect protein:protein interactions in living cells (Xu et al., 1999, Proc Natl Acad Sci USA, 96, 151-156). When HLS-5 and Ubc9 constructs were co-expressed in COS cells a positive BRET ratio was observed, demonstrating that these proteins do indeed interact within cells (Figure 1b). However, this interaction may well be transient as the two molecules could not be co-immunoprecipitate (data not shown). A likely explanation for this is that most other published targets of UBC9, apart from RanGAP1, appear to require above stoichiometric amounts of UBC9 (often µM range), and even then are modified only marginally. Of note, two hybrid interactions between UBC9 and SUMO targets have been reported in multiple studies but stable interaction with recombinant UBC9 in the absence of cell extracts (e.g. reticulocyte lysate) has been shown only for RanGAP1 and the E3 ligases RanBP2, Siz1 and Siz2. Also it has been suggested that the binding to UBC9 is dependant on its ability to bind simultaneously a SUMO thioester charged UBC9 and a target, acting as a bridge.

Regions important for mediating the interaction between HLS-5 and PIAS1 were also defined by yeast 2 hybrid assays, and Figure 1c shows that the amino terminal portion of HLS-5 consisting of the B box and coiled-coil motifs was required for this association. In reverse, the C-terminal end of PIAS1, distal to the RING-like domain responsible for its E3 Sumo ligase activity, was involved in the interaction with HLS-5 (data not shown).

To demonstrate that the full length proteins associated in mammalian cells, COS cells were transiently transfected with HLS5 and PIAS1 then immunoprecipitated; however, no association could initially be detected in this assay (Figure 1d, first 6 transfections). As PIAS1 mainly is a nuclear protein (Tan et al., 2000, Mol Endocrinol, 14, 14-26), and HLS-5 is primarily cytoplasmic, the failure of these molecules to interact may be caused by subcellular separation. Since GATA-1 promotes nuclear re-localization of HLS-5 (see below and Figure 3b) it was included in co-transfections with HLS5 and PIAS1. Figure 1c, last 2 transfections, shows that HLS-5 and PIAS1 could co-immunoprecipitate, when GATA-1 was present. It is postulated that the redistribution of HLS-5 induced by GATA-1 enabled nuclear access and binding to PIAS1 or that GATA-1, HLS-5 and PIAS1 form a protein complex required for this interaction.

We also observed a accumulation of HLS-5 caused by MG132 (Figure 3C) and this accumulation also, as in the case of adding GATA-1, resulted in CO-IP of PIAS1 with HLS-5 (data not shown). Interestingly, the co-immunoprecipitation and co-localization of HLS-5 with PIAS1 was increased by the presence of MG132, suggesting that this interaction may be more efficient around the nuclear bodies. Also, COS7 cells co-expressing Myc-tagged HLS-5 or a mutant form of HLS-5 lacking its Ring finger (ΔN61) and HA-tagged SUMO1 were analysed in the presence or absence of MG132. Fig 6A shows a SUMOylated substrate detected with an anti-HA antibody which was specific to the HLS-5 lane expressing full length HLS-5 in the presence of MG132, but absent when the Ring finger was mutated or when MG132 was absent. This suggests that the interaction is dependent on a protein-protein interaction via the RING finger or that HLS-5 has a SUMO E3 Ligase activity.

### EXAMPLE 2 HLS-5 IS SUMOYLATED AND AFFECTS SUMOYLATION OF GATA-1 AS WELL AS GLOBALLY

Since HLS-5 was able to interact with Ubc9 and PIAS1, the possibility that HLS-5 is sumoylated was then examined. Like other RBCC/TRIM family members such as PML (Duprez et al., 1999, J Cell Sci., 112, 381-393; Kamitani et al., 1998, J Biol. Chem, 273, 26675-26682), HLS-5 has several ΨKxE/D consensus sumoylation sites (Figure 2a). To determine if HLS-5 was able to bind SUMO-1, the yeast two-hybrid system was employed using several HLS-5 deletion mutants. This assay showed that HLS-5 could interact with SUMO-1, and that the association was dependent upon the coiled-coil domain of HLS-5, containing two potential sumoylation motifs (Figure 2b). Interestingly, this interaction was specific for SUMO-1 as neither SUMO-2, nor SUMO-3 bound HLS-5 in yeast (Figure 2c).

It was unclear from these yeast two-hybrid assays whether HLS-5 was interacting non-covalently with SUMO-1, or whether SUMO-1 was conjugated *in vivo.* The results displayed in Figure 2D show that in addition, cotransfection and immunoblotting demonstrated SUMO-1 was conjugated to HLS-5 in mammalian cells, that this reaction was enhanced by the presence of UBC9 and that PIAS1 did not serve as an E3 for HLS-5, as the SUMOylation products of HLS-5 were reduced by its presence.

Several RING finger-containing proteins have been shown to act as E3 ligases for SUMO-1 (Jackson, 2001, Genes Dev, 15, 3053-3058). To investigate whether HLS-5 was a functional E3 ligase, SUMO-1 and HLS-5 were co-expressed in COS cells and total protein sumoylation ascertained by immunoblotting. Figure 5B, middle lanes, shows that HLS-5 had a major impact upon global SUMOylation. The overall sumoylation was reduced in the presence of HLS-5, although some proteins displayed elevated sumoylation (Figure 6A). Significantly, the levels of SUMO-1 itself were reduced by >95% (Figure 5B), In addition co-expression of HLS-5 and PIAS1 resulted in an almost complete elimination of PIAS1 from the cell (Figure 5b, first 2 transfections). HLS-5 also reduced the levels of Ubc9, although not to the same extent as SUMO-1 and PIAS1 (Figure 5B, lane 5 & 6). We then investigated which domain of HLS-5 was involved in the elimination of PIAS1 protein expression. As shown in Figure 5c, there was reduced expression of PIAS1 with the HLS-5 (ΔN150) deletion mutant, but the levels approached to control levels with the HLS-5 (ΔN267), indicating that the B Box and Coiled Coil domain were required for this HLS-5 function, consistent with the PIAS1 interaction domains in the yeast two hybrid experiments (Figure 1C). Thus, HLS-5 altered global protein SUMOylation and reduced intracellular content of SUMO-1, PIAS1 and Ubc9, key components of the SUMOylation machinery and this action is accomplished partly through its B Box & Coiled Coil domain.

### EXAMPLE 3 SUBCELLULAR LOCALIZATION OF HLS-5 IS AFFECTED BY SUMO-1 AND MG-132

Data presented above show that HLS-5 can associate with Ubc9, PIAS1 and SUMO-1. As HLS-5 is located principally within cytoplasmic granules, the effects of Ubc9, PIAS1, MG-132 and SUMO-1 on HLS-5 compartmentalization were investigated. Ubc9 is present throughout the cell and did not influence the subcellular localization of HLS-5; similarly, the nuclear protein PIAS1 did not initiate relocation of HLS-5 protein, but as seen by western analysis and confocal microscopy lead to elimination of its protein expression (Figure 5A & 5B). However, when the SUMO-1 and HLS-5 were co-expressed, there was a remarkable increase in the amount of HLS5 appearing in the nucleus (Figure 3A). Therefore SUMO1 promoted translocation of HLS-5 from cytoplasmic granules into the nucleus. This translocation was also observed in the presence of MG-132 (Figure 6C).

To examine the biological effects of Ubc9, PIAS1 and SUMO-1 on HLS5-induced apoptosis, COS cells and HeLa cells were co-transfected with these molecules and cell death monitored. Significantly in Hela cells, Ubc9 was able to suppress apoptosis caused by HLS5, from 60% of cells to only 12%; moreover a dominant negative Ubc9 which is unable to participate in sumoylation was also capable of reducing HLS-5-promoted cell death to 4%. However, the effects of SUMO-1 on cell death induced by HLS5 were less pronounced. These results indicate that SUMOylated HLS-5 may have a less pronounced effect on apoptosis than the unmodified protein.

### EXAMPLE 4 HLS-5 AFFECTS GATA-1 SYMOYLATION AND TRANSACTIVATION

Recently, GATA-1 has been shown to be sumoylated and its activity repressed by this modification (Collavin *et al.,* 2004, *supra*). Since HLS5 can interact with the sumoylation machinery, the relationship with GATA-1 was examined. To determine if GATA-1 could influence the subcellular localization of HLS5, COS cells were transiently transfected and examined by confocal microscopy. Co-expression of the two proteins resulted in the majority of HLS-5 protein moving into the nucleus, although some residual granular cytoplasmic staining was observed (Figure 3B). GATA-1 and HLS5 were uniformly distributed throughout the nucleus, except for discrete regions where both proteins were excluded.

The interaction between GATA-1 and HLS5 was then examined biochemically by transfecting COS cells with myc-tagged HLS-5 and GATA-1, followed by immunoprecipitation and immunoblotting. While GATA-1 was detected in HLS-5 immunoprecipitates, this interaction was disrupted in the absence of isopeptidase inhibitors N-ethylmaleimide (NEM) and iodoacetamide (IAA), suggesting that thiol bonds are important for this association or that only sumoylated or ubiquitinated species can interact (Figure 4A). This result indicates that HLS-5 and GATA-1 can exist in a complex dependent on post-translational modification of either or both proteins.

COS cells were then transfected with various combinations of GATA-1, SUMO-1, PIAS1 and HLS-5, then sumoylated GATA-1 was identified by immunoblotting. Figure 4B shows that a slower migrating GATA-1 protein was detected, particularly in lysates containing SUMO-1 and PIAS1; indicating GATA-1 was sumoylated. As this higher molecular weight GATA-1 species was much less abundant when only GATA-1 and SUMO-1 were combined, it appears that PIAS1 was the limiting factor in the reaction. Significantly, addition of HLS-5 resulted in an almost complete loss of the larger GATA-1 species (Figure 4B).

To confirm that the larger form of GATA-1 was sumoylated GATA-1, transfected COS cells were immunoprecipitated followed by immunoblotting to identify HA-tagged SUMO-1. The data presented in Figure 4b demonstrate that the slower migrating form of GATA-1 was indeed sumoylated GATA-1. Reprobing the blot with anti-SUMO-1 antibodies confirmed that the sumoylated protein was GATA-1 (data not shown). A small amount of sumoylated GATA-1 was observed when GATA-1 and SUMO-1 were co-transfected; importantly, HLS5 reduced the levels of sumoylated GATA-1 by 70% (Figure 4C). Similarly, the markedly elevated levels of sumoylated GATA-1 detected in the presence of PIAS1 were suppressed almost 80% by HLS-5 (Figure 4c). It is noteworthy that sumoylation was only detected in the presence of isopeptidase inhibitors NEM and IAA. It was concluded from these experiments that GATA-1 sumoylation was inhibited by HLS-5.

The effect of HLS-5 on sumoylated GATA-1 transactivation was then examined using the MIα promoter and luciferase reporter assays. Initial experiments revealed that increasing SUMO-1 levels in transfected COS cells resulted in an increase in GATA-1 transactivation; addition of PIAS1 enhanced GATA-1 activity further (Figure 4D). These observations contrast with the inhibitory effects of GATA-1 sumoylation on transactivation reported by Collavin *et al.* (2004, *supra*). Significantly, co-expression of HLS5 with SUMO-1 resulted in decreased GATA-1 activity (Figure 5D). Furthermore, HLS5 suppressed GATA-1 transactivation in the presence of both SUMO-1 and PIAS1. Comparable data were obtained using α-integrin promoter (data not shown). Taken together, the data indicate that HLS5 inhibits GATA-1 activity by interfering with the sumoylation of this key erythroid transcription factor. These observations are consistent with a modulatory role for HLS5 in sumoylation.

Since putative cis-acting enhancer elements were expected to exist in the cloned promoter region from gene of interests, the luciferase activity observed during the reporter assay more closely resembles the actual regulation of this genes within human cells. As a means of measuring promoter response in cells and for its use in high throughput screening, the luciferase assay was used in the context of reporter vector pGL3-Basic (Promega). Stable cell lines were derived from human HeLa cells with chromosomal integration of a luciferase reporter construct regulated by HLS-5's response element and the puromycin resistance gene used as a selectable marker. These clonal cell line was obtained by cotransfection of pH5-34-luc (Fig 7A and B represent diagram of the construct elements) and pBabe-Puro followed by Puromycin selection at 1 ug/ml and isolation of single clones. The promoter reporter vector contains a 961 Bp insert (SEQ ID NO:9), corresponding to the 5'-flanking sequence upstream of the initiation codon of HLS-5. This insert was placed upstream of the promega luciferase reporter gene (pGL3-Basic). The cells used as controls and generated were: Hela and Hela-Puro (containing the selectable marker alone), Clone A1, Clone B2, Clone F2, Clone C7 and Clone C27 (which were HLS-5 promoter expressing clones).

EPO, PMA, INF-Gamma, IL-6, and luciferase activity was measured in each clone (Fig 7C). These experiments revealed clones which had a capacity for both induction and repression, but with higher background (clone A1) and average induction levels of around 3-fold (Clones B2). The clone C27 had low background and a nearly 20-fold induction, which is more than sufficient and reproducible. This allows less false positive and more efficient screening.

Materials used for the culture and maintenance of the clones and cell lines were:
DMEM with Phenol Red (Thermo, Cat. # 50-126-PB)
Foetal Bovine Serum (Gibco, Cat. # 10099-141)
Pen/Strep (Gibco, Cat. # 15070-063)
Puromycin Control (Sigma, Cat. # P8833)
Dual-Luciferase Reporter Assay System (Promega, cat.# E1910)
Trypsin-EDTA (Gibco, Cat. # 25300-062)

For controlled inductions these products were used at the following final concentrations:

| Inducer Name | Company | Catalog # | Conc. Used |
|---|---|---|---|
| PMA | Sigma | | 100 nM |
| DMSO | Sigma | D5879 | 1.5% |
| EPO | EPREX | EPREX 4000 | 10U/ml |
| INFgamma | Roche | | 100U/ml |
| IL-6 | Sigma | I9646 | 10 ng/ml |

The procedure was modified from the manufacturer's instructions for the luciferase assay (Promega) in a 6-well culture plate. Two days before collection of samples, 1-3x10⁵ cells were plated in 2 ml of their normal growth media (DMEM containing 10 % serum with antibiotics and puromycin). Cells were always plated in triplicate for each condition. This amount yielded 50-80% confluence on the day of treatment with induction agents. One group of each cell line was used for treatment and the other group for control. For each well, the inducing agent was added and incubated for 18h, the cells were incubated overnight at 37°C in a CO₂ incubator.

After incubation, cells were trypsinised and all material was collected in order to completely remove the media from the culture plates. The collected material was centrifuged at 900 rpm and resuspended in 1 ml of PBS. The material was then transferred to a clean 1.5-ml micro centrifuge tube and after centrifugation , the cells were lysed by adding 300 µl of passive lysis buffer (Promega, Luciferase Assay System) with the content of each well. The product was kept on ice and eventually stored at -20°C. After 3 cycles of freeze, thaw and vortex, the lysates were clarified by 15 min centrifugation at 4°C and the supernatant was used in subsequent luciferase assays. For the luciferase assay, 50 µl of cleared lysate was used per well, with 50 µl of LAR II in a 96-well plate. After protein quantification (BioRad), the luciferase activity was measured as light units/µg of protein relative to the untreated or vector only controls.

## Claims

1. A HLS-5 polypeptide, comprising or consisting of the sequence set out in SEQ ID NO: 6.

2. A polynucleotide encoding for a HLS-5 polypeptide of claim 1.

3. The polynucleotide of claim 2, comprising the nucleotide sequence set out in SEQ ID NO: 5.

4. A method of regulating sumoylated GATA-1, comprising the step of administering a HLS-5 polypeptide comprising the amino acid sequence shown in SEQ ID NO: 2, SEQ ID NO: 4, SEQ ID NO: 6 or a functional fragment thereof.

5. A method of claim 4, wherein a HLS-5 polypeptide of claim 1 is administered.

6. A method of regulating sumoylated GATA-1 in vitro, comprising the step of administering a HLS-5 polypeptide comprising the amino acid sequence shown in SEQ ID NO: 2, SEQ ID NO: 4, SEQ ID NO: 6 or a functional fragment thereof.

7. A method of claim 6, wherein a HLS-5 polypeptide of claim 1 is administered.

8. A HLS-5 polypeptide of claim 1 or a polynucleotide of one of claim 2 or 3 for use in treatment of Parkinson's disease, diabetes, Huntington's disease, neuronal intranuclear inclusion disease, Alzheimer's disease, polyglutamine disease, HIV infection.


SEQUENCE LISTING

<110> BioPharmica Ltd.

<120> Sumoylation control agent and uses thereof

<130> P555508EP/PC-Zie

<140> 06790340.1
<141> 2006-10-06

<150> US20050725171
<151> 2005-10-07

<150> WO2006AU01470
<151> 2006-10-06

<160> 8

<170> PatentIn Ver. 2.1

<210> 1
<211> 3687
<212> DNA
<213> Mus musculus

<400> 1
   tcagcaggga caacgcccgc aggctaggca tgaaggcggc gacaccggtg gtggtgacgg 60
   cggctgctcc tgcgatggag ccgggccctt ctgtgtcccc ggggccttcg cgctccttca 120
   aagaggagct gctgtgtgcc gtgtgctacg acccgttccg cgacgcagta actctgcgct 180
   gtggccacaa cttctgccgc cggtgcgtga gcggctgctg ggaggtgcag acgacgccct 240
   cgtgtccggt gtgcaaggaa cgagcggtgc ccggggagct gcgcaccaac cacacgctca 300
   acaacctggt ggagaccttg ctgcgcgagg aggctgaggg cgcgcgctgg accggtcgcc 360
   ggtccccgcg cccctgccgt gcgcaccgtg ccccgctcac gctcttctgc ctggaggaca 420
   aggagctgct gtgctgtgct tgccaggccg acgcccggca ccaagagcat cgtgtgcagc 480
   ccatcaagga cactgcgcaa gacttccggg ccaagtgtaa gaacatggag catgtattgc 540
   gagagaaagc caaggccttc tgggccctga ggcgcaccta tgaggccatt gccaagcaca 600
   atgaggtgca aacgacttgg ctggaaggcc gcatccggga tgagtttgac aagctccgtg 660
   atttcctgag ggtggaggaa caggccacct tggatgccat gaaggaagag agcagaaaga 720
   agcacctgca ggctgaggag aagatgaagc agctagcaga acagaccgag gcgctggctc 780
   gggagattga gcgtctgcag atggagatga aggaagatga catgaccttc ctcatgaaac 840
   acaagagccg aaaacgccgg ctcttctgca ccgtggagcc agctcctctc cagcctggct 900
   tgctaatgga tgcatgcaag tatctggagt ccctgcagta ccgagtctgg aagaagatgc 960
   ttggatccgt tgagtctgtg cccttcagct tggatcccaa cacagctgct ggctggctca 1020
   aagtggctga tgacctcacg agtgtcatca accatggcta ccgcgtgcaa gtggagaatc 1080
   cagagcgctt ctcctcggca ccctgcctgc taggctctca agtgttctcc aagggctccc 1140
   actcttggga ggtggatgtg ggtggcctgc caacctggcg agtgggtgtg gttcgggtgc 1200
   aggcacatgc acaggcgcag gctcaggctg acgtaggtgg tgaaggccac tcacacagct 1260
   gctaccatga tacacgctca ggcttctggt acctgtgccg cacgcagggt gtggatggag 1320
   accactgcat gacttccgac actgccacag cccctctggt ccaggccatg ccgcgccgtc 1380
   tgcgtgtgga gctggagtgt gaggagggtg agctatcctt ctatgactct gagcgccact 1440
   gccatctgta taccttccat gcccactttg gggaggtgag gccctacttc tacctgggag 1500
   cctctcgagg tgacggtccc ccggaacctc tgcgtatctg ccacctgcgt gtctccatca 1560
   aagaagagct ggacatctga gctgcccacc cctgacacat gcagcatact atatcctgtc 1620
   ttagcttttc tgtagctcca aagttaggag ccacccagga ggtgcttggc tgagcctagg 1680
   ctctgtctac agtcatgcta ccttcaggat gtgggttttc tgttcttgga ttgctggtat 1740
   actgttttct ttgtaggatg gcattataat gtaggtgtag actattttta gagatgatga 1800
   agccagccta tcaggagatg cctcttattg agtctatttg tcatttatgt ttcccaggaa 1860
   gaggtccttg tcaggccaca cagggaagcc ccaggatggt ttgagacaag agtgggcaaa 1920
   gccttcacct aggctctccc accccagtag gacaagttag gtattggcca gcctcactga 1980
   gctatgcatg gctttagcat ctggtctgaa ccaggagtcc ccgtgttggt tcctaggaca 2040
   ggattgtcct tgactctctc tgtgggaaca ctgtagggtg tgaggtactc tggaatacag 2100
   ctcagagttg tgggtgtctc aggaaaggca gctccagggc ctttggcagt ggttaaggac 2160
   tgaccagcct caagttagtg ccatagaggc caaagcacca gaatgcagtg agtgagaaga 2220
   cagctagtgc tgggttggaa catgagcccc actctgttac tcagtctttc ggcacggaat 2280
   cgacactggt tggtcacctc atgctttgaa cgtttcctct ggaattgtcc agttttctag 2340
   aacacttttt aaacctgtgt ttccacatct gtgatttgac actagtcctt ggaaatcact 2400
   ggaggaaggt atgagaagga gcctcagaga atgctgcttc agtcagtgac tgttggtctg 2460
   ggagacgagt cctggccttg ctggccgttg tcttcattgg cttccacctg cctgtccttg 2520
   ctctgctctc tgcagattcc acccctttcc tttatgtgtc cctctgcctt ttctttctca 2580
   gtcatgcctg tagatggagt ctaattgcca actaaccaaa tcccaagaga tgttgtaagg 2640
   aaaaaatacc atctcagggg tgccctgtca tcgtcaagac ctaggaccta gcatcccaat 2700
   ttcagcctgc acccctcatt acataagact tgttttaaac cacgccgatt acccactaat 2760
   tggctctaaa tgggtcatgt gcacttgtgg attatctaac aagtggagac acagaagaaa 2820
   ccctggtgca ggccaggccg ggagcaggga cagtgttggc aagccagctt gtgagtgtca 2880
   gatgcttggg caccacggat gtgaaaggtg cgcctggtgc aatgtatgtg tttggttaaa 2940
   gaaactctct gaaattactg ttataataag tttttaaaag tttttctttt ctttttaatt 3000
   ttcaccttaa ctcttaaata gggtaatttc aatgacctag actcttagaa aaaatttgac 3060
   ttaccccaca actgacatgt ttctttcaga gcttttgtaa acacaaattc ctagtgtaac 3120
   ttgtacctga tctgtccttc ccattgtaag attccatcgt gtgcagtgaa tgtgctgtgc 3180
   aacttgttag ttgatggaca tttggcttgt tgcagtgtgt ttagctgtta tgagttctgc 3240
   tgctatgaaa tttgtgtgca agttttgtgt ggctgtgtct cttgattttc tgtcatatac 3300
   cttggagtct gttttccagg tcatatgata attattttaa gccctttggg gacttgccaa 3360
   gctgtttcta agagagccgt cccattttct actcccaccg gcagcaggtg agggctggtt 3420
   tgtaacgaat tctctctgcc ctcttaagct gaggaagctg gagtaggtct catttgccct 3480
   gtagttgcga tctctgatgg ctggggagca tctttcctca tgtttgctgt gtatctgctt 3540
   cagagacttc agggtgtttg cccattgggt tgtctgacct tttattatga aggtttacaa 3600
   gtttgttatg cattctagat aaaagttcct ttgtgtcaga tgaatcacat aaaaattttc 3660
   ctcctaattc aaaaaaaaaa aaaaaaa 3687

<210> 2
<211> 501
<212> PRT
<213> Mus musculus

<400> 2
Met Glu Pro Gly Pro Ser Val Ser Pro Gly Pro Ser Arg Ser Phe Lys
1               5                   10                  15

Glu Glu Leu Leu Cys Ala Val Cys Tyr Asp Pro Phe Arg Asp Ala Val
            20                  25                  30

Thr Leu Arg Cys Gly His Asn Phe Cys Arg Arg Cys Val Ser Gly Cys
        35                  40                  45

Trp Glu Val Gln Thr Thr Pro Ser Cys Pro Val Cys Lys Glu Arg Ala
    50                  55                  60

Val Pro Gly Glu Leu Arg Thr Asn His Thr Leu Asn Asn Leu Val Glu
65                  70                  75                  80

Thr Leu Leu Arg Glu Glu Ala Glu Gly Ala Arg Trp Thr Gly Arg Arg
                85                  90                  95

Ser Pro Arg Pro Cys Arg Ala His Arg Ala Pro Leu Thr Leu Phe Cys
            100                 105                 110

Leu Glu Asp Lys Glu Leu Leu Cys Cys Ala Cys Gln Ala Asp Ala Arg
        115                 120                 125

His Gln Glu His Arg Val Gln Pro Ile Lys Asp Thr Ala Gln Asp Phe
    130                 135                 140

Arg Ala Lys Cys Lys Asn Met Glu His Val Leu Arg Glu Lys Ala Lys
145                 150                 155                 160

Ala Phe Trp Ala Leu Arg Arg Thr Tyr Glu Ala Ile Ala Lys His Asn
                165                 170                 175

Glu Val Gln Thr Thr Trp Leu Glu Gly Arg Ile Arg Asp Glu Phe Asp
            180                 185                 190

Lys Leu Arg Asp Phe Leu Arg Val Glu Glu Gln Ala Thr Leu Asp Ala
        195                 200                 205

Met Lys Glu Glu Ser Arg Lys Lys His Leu Gln Ala Glu Glu Lys Met
    210                 215                 220

Lys Gln Leu Ala Glu Gln Thr Glu Ala Leu Ala Arg Glu Ile Glu Arg
225                 230                 235                 240

Leu Gln Met Glu Met Lys Glu Asp Asp Met Thr Phe Leu Met Lys His
                245                 250                 255

Lys Ser Arg Lys Arg Arg Leu Phe Cys Thr Val Glu Pro Ala Pro Leu
            260                 265                 270

Gln Pro Gly Leu Leu Met Asp Ala Cys Lys Tyr Leu Glu Ser Leu Gln
        275                 280                 285

Tyr Arg Val Trp Lys Lys Met Leu Gly Ser Val Glu Ser Val Pro Phe
    290                 295                 300

Ser Leu Asp Pro Asn Thr Ala Ala Gly Trp Leu Lys Val Ala Asp Asp
305                 310                 315                 320

Leu Thr Ser Val Ile Asn His Gly Tyr Arg Val Gln Val Glu Asn Pro
                325                 330                 335

Glu Arg Phe Ser Ser Ala Pro Cys Leu Leu Gly Ser Gln Val Phe Ser
            340                 345                 350

Lys Gly Ser His Ser Trp Glu Val Asp Val Gly Gly Leu Pro Thr Trp
        355                 360                 365

Arg Val Gly Val Val Arg Val Gln Ala His Ala Gln Ala Gln Ala Gln
    370                 375                 380

Ala Asp Val Gly Gly Glu Gly His Ser His Ser Cys Tyr His Asp Thr
385                 390                 395                 400

Arg Ser Gly Phe Trp Tyr Leu Cys Arg Thr Gln Gly Val Asp Gly Asp
                405                 410                 415

His Cys Met Thr Ser Asp Thr Ala Thr Ala Pro Leu Val Gln Ala Met
            420                 425                 430

Pro Arg Arg Leu Arg Val Glu Leu Glu Cys Glu Glu Gly Glu Leu Ser
        435                 440                 445

Phe Tyr Asp Ser Glu Arg His Cys His Leu Tyr Thr Phe His Ala His
    450                 455                 460

Phe Gly Glu Val Arg Pro Tyr Phe Tyr Leu Gly Ala Ser Arg Gly Asp
465                 470                 475                 480

Gly Pro Pro Glu Pro Leu Arg Ile Cys His Leu Arg Val Ser Ile Lys
                485                 490                 495

Glu Glu Leu Asp Ile
            500

<210> 3
<211> 4196
<212> DNA
<213> Homo sapiens

<400> 3
   ctcgggtcgc gggcgttggc agccgggcgg gtgggagggg ccggagcaaa agttccgggc 60
   gcccgagccg gctgctcgtg ccatggagcg gagtcccgac gtgtccccgg ggccttcccg 120
   ctccttcaag gaggagttgc tctgcgccgt ctgctacgac cccttccgcg acgcagtcac 180
   tctgcgctgc ggccacaact tctgccgcgg gtgcgtgagc cgctgctggg aggtgcaggt 240
   gtcgcccacc tgcccagtgt gcaaagaccg cgcgtcaccc gccgacctgc gcaccaacca 300
   caccctcaac aacctggtgg agaagctgct gcgcgaggag gccgagggcg cgcgctggac 360
   cagctaccgc ttctcgcgtg tctgccgcct gcaccgcgga cagctcagcc tcttctgcct 420
   cgaggacaag gagctgctgt gctgctcctg ccaggccgac ccccgacacc aggggcaccg 480
   cgtgcagccg gtgaaggaca ctgcccacga ctttcgggcc aagtgcagga acatggagca 540
   tgcactgcgg gagaaggcca aggccttctg ggccatgcgg cgctcctatg aggccatcgc 600
   caagcacaat caggtggagg ctgcatggct ggaaggccgg atccggcagg agtttgataa 660
   gcttcgcgag ttcttgagag tggaggagca ggccattctg gatgccatgg ccgaggagac 720
   aaggcagaag caacttctgg ccgacgagaa gatgaagcag ctcacagagg agacggaggt 780
   gctggcacat gagatcgagc ggctgcagat ggagatgaag gaggacgacg tttcttttct 840
   catgaaacac aagagccgaa aacgccgact cttctgcacc atggagccag agccagtcca 900
   gcccggcatg cttatcgatg tctgcaagta cctgggctcc ctgcagtacc gcgtctggaa 960
   gaagatgctt gcatctgtgg aatctgtacc cttcagcttt gaccccaaca ccgcagctgg 1020
   ctggctctcc gtgtctgacg acctcaccag cgtcaccaac catggctacc gcgtgcaggt 1080
   ggagaacccg gaacgcttct cctcggcgcc ctgcctgctg ggctcccgtg tcttctcaca 1140
   gggctcgcac gcctgggagg tggcccttgg ggggctgcag agctggaggg tgggcgtggt 1200
   acgtgtgcgc caggactcgg gcgctgaggg ccactcacac agctgctacc acgacacacg 1260
   ctcgggcttc tggtatgtct gccgcacgca gggcgtggag ggggaccact gcgtgacctc 1320
   ggacccagcc acgtcgcccc tggtcctggc catcccacgc cgcctgcgtg tggagctgga 1380
   gtgtgaggag ggcgagctgt ctttctatga cgcggagcgc cactgccacc tgtacacctt 1440
   ccacgcccgc tttggggagg ttcgccccta cttctacctg gggggtgcac ggggcgccgg 1500
   gcctccagag cctttgcgca tctgcccctt gcacatcagt gtcaaggaag aactggatgg 1560
   ctgagctggc ccggggctgc cccggtcttg tgccacagca ctgttttctt tctgccctct 1620
   tcctaatgcc cacactgctt gggcactatt gcgcccctgc ctccttgcca ggctcttcct 1680
   cctgtcctgc ctggtccttt tccatgactc caggctgtgc ctctctccat gtttggtccc 1740
   ttctgtgccc atggtcagga gctattcggg tggcacctcg ctggccaggc tctcccgagt 1800
   cgtggcacct ccacaatgtg aattttctga atccctattc caggatttct gggaataatg 1860
   tttacttcta gaatgggcct gttgtaaacc atctcatcga ggtgtggtaa agccattgga 1920
   tgaggagggg actgccatgg aaaggagagt ttgttactta cggttctgag aggaggggcc 1980
   acataggaaa gccccacggt gggtcagaag gcggaaggag ggaggggaac gtgtgggcaa 2040
   gagacttcct ctggtttcct caggaggaaa tgggcaaggc agagtaagca ggggagacag 2100
   gtttaagggt agctggcttg agtaatttca gtggctctca ggataggggc tgcccttttt 2160
   gtctgatacc tggccccggg atagtcagga caggtgaatg ttggcctggg gtgtgacagc 2220
   cctgggagag ccatgtgaag gaggcagctg gcgccatcgc tccggattag ttggtttcca 2280
   taggaaaggc atgctttcag ccagatgctt gccatctcta gggattgggg gattggctag 2340
   cctgggagga tcagtctgtc caggtcagcg aggccccaga taccagagca tcaagagtac 2400
   aggaaataca gttaatgcag ggcctctgtg tggctggatc ctccgtctcc atcagatcag 2460
   ctctgattga tctattcttg cacgatttcc tctgaacaca gggttccaga gtacttaaac 2520
   acaacatttt ttaaatcgtg atttcggcct atttccttgc caggcctgtt tccccaccag 2580
   gaaatgagat aggaggactg gatgaggatg tcctgttata gttgctgtgg aggaagttcc 2640
   tctggttaat tctcatcagc gtctgcagaa aagaaggaaa gagggcaccc ttttcagttg 2700
   ggaagaaagg agaggggtgg cgccatggac gtggccctaa acgctgtggg agagggaaga 2760
   ggaggctggg cctcgctgcc ctcttgtctc tgctgacttc agcctggtca tgcttgctct 2820
   gccacttgcg atttcatccc taatttcttc ctccaccatg cctgcagact tttccctggg 2880
   cttgtttttt ctcgcacatc tctgaagagt ttttaatctt cagctcatca tgtcccagga 2940
   agtggcatca taaaaggaaa tatttttttt tcctaggagc agtgttaaaa tctgggtcac 3000
   attcctgacc aaggacagca tcctgccttc tgcccatccc cttcagttca caaaagctga 3060
   cattttaaac aaatcatgac tcacacgtat taattggtta taaatatgtt gtgtacactg 3120
   gttagataaa acttaaggcc acaaggaggg cccaggtagg cgatgtcagt gtgtgaaggg 3180
   gctggattgg gcgtggtgag gatgttggca aaccagtgca tgcacctggt tggaagatgc 3240
   tcagcctcac aaaagctcca agccctttgg gagccaaagt gtctgagagt gtgaccctct 3300
   cctgtaaagt atttatccca cccattaata taatttctgt ataataaact tgacctgaaa 3360
   ttatttcatt ctttatatta aacttttaaa aatgtttttt attttcacct tagatatggg 3420
   aagagttttt tttttttttt ttttttttta acaggataac ttgagcaggc taggcctctt 3480
   aaaaaaaaat ttgagctaaa actcattttt cttttggcat tttcttttca atgttcttat 3540
   aagcaaagtt catccatgtt gtagcatgtg ttcaacttta ttttttcatc gggtaatatt 3600
   ccattgtatg gaatggtagt actacatttt atttatcatg catcgattgg tggacatttg 3660
   gatcgtttct acttcttgac tattatacat aatgctgcta ggaacttttg tgtatgagtt 3720
   tttgtgtgga catatgtttt catttctctt tggtatatgc ctgggagcag atttgctgga 3780
   tcatatgaaa ctctatttaa cccttgaggg actcccaaac tgttttccaa tgtggtgaca 3840
   caattttata tcccatcaac agggcatgag ggttctgatg actccacatc cctcagtgct 3900
   ttttattatc tatcttttaa cttagccatc ctagtagggg taatgtggca tctcattgtg 3960
   attttggttt gcatttccct gatggcgaat gatattgagc atcttttcat gagcttattg 4020
   gccatttgca tatcttctta ggacagctat ctttagatca cttgctcatt ttttaattgg 4080
   gttatttgtc tttttattat tgagttgtaa gagtcctttt atagcctggc acaagtccct 4140
   ttaactggta tatgattata aaatttttct ccgtgagctg tttcatttcc ttgatg 4196

<210> 4
<211> 493
<212> PRT
<213> Homo sapiens

<400> 4
Met Glu Arg Ser Pro Asp Val Ser Pro Gly Pro Ser Arg Ser Phe Lys
1               5                   10                  15

Glu Glu Leu Leu Cys Ala Val Cys Tyr Asp Pro Phe Arg Asp Ala Val
            20                  25                  30

Thr Leu Arg Cys Gly His Asn Phe Cys Arg Gly Cys Val Ser Arg Cys
        35                  40                  45

Trp Glu Val Gln Val Ser Pro Thr Cys Pro Val Cys Lys Asp Arg Ala
    50                  55                  60

Ser Pro Ala Asp Leu Arg Thr Asn His Thr Leu Asn Asn Leu Val Glu
65                  70                  75                  80

Lys Leu Leu Arg Glu Glu Ala Glu Gly Ala Arg Trp Thr Ser Tyr Arg
                85                  90                  95

Phe Ser Arg Val Cys Arg Leu His Arg Gly Gln Leu Ser Leu Phe Cys
            100                 105                 110

Leu Glu Asp Lys Glu Leu Leu Cys Cys Ser Cys Gln Ala Asp Pro Arg
        115                 120                 125

His Gln Gly His Arg Val Gln Pro Val Lys Asp Thr Ala His Asp Phe
    130                 135                 140

Arg Ala Lys Cys Arg Asn Met Glu His Ala Leu Arg Glu Lys Ala Lys
145                 150                 155                 160

Ala Phe Trp Ala Met Arg Arg Ser Tyr Glu Ala Ile Ala Lys His Asn
                165                 170                 175

Gln Val Glu Ala Ala Trp Leu Glu Gly Arg Ile Arg Gln Glu Phe Asp
            180                 185                 190

Lys Leu Arg Glu Phe Leu Arg Val Glu Glu Gln Ala Ile Leu Asp Ala
        195                 200                 205

Met Ala Glu Glu Thr Arg Gln Lys Gln Leu Leu Ala Asp Glu Lys Met
    210                 215                 220

Lys Gln Leu Thr Glu Glu Thr Glu Val Leu Ala His Glu Ile Glu Arg
225                 230                 235                 240

Leu Gln Met Glu Met Lys Glu Asp Asp Val Ser Phe Leu Met Lys His

245                 250                 255

Lys Ser Arg Lys Arg Arg Leu Phe Cys Thr Met Glu Pro Glu Pro Val
            260                 265                 270

Gln Pro Gly Met Leu Ile Asp Val Cys Lys Tyr Leu Gly Ser Leu Gln
        275                 280                 285

Tyr Arg Val Trp Lys Lys Met Leu Ala Ser Val Glu Ser Val Pro Phe
    290                 295                 300

Ser Phe Asp Pro Asn Thr Ala Ala Gly Trp Leu Ser Val Ser Asp Asp
305                 310                 315                 320

Leu Thr Ser Val Thr Asn His Gly Tyr Arg Val Gln Val Glu Asn Pro
                325                 330                 335

Glu Arg Phe Ser Ser Ala Pro Cys Leu Leu Gly Ser Arg Val Phe Ser
            340                 345                 350

Gln Gly Ser His Ala Trp Glu Val Ala Leu Gly Gly Leu Gln Ser Trp
        355                 360                 365

Arg Val Gly Val Val Arg Val Arg Gln Asp Ser Gly Ala Glu Gly His
    370                 375                 380

Ser His Ser Cys Tyr His Asp Thr Arg Ser Gly Phe Trp Tyr Val Cys
385                 390                 395                 400

Arg Thr Gln Gly Val Glu Gly Asp His Cys Val Thr Ser Asp Pro Ala
                405                 410                 415

Thr Ser Pro Leu Val Leu Ala Ile Pro Arg Arg Leu Arg Val Glu Leu
            420                 425                 430

Glu Cys Glu Glu Gly Glu Leu Ser Phe Tyr Asp Ala Glu Arg His Cys
        435                 440                 445

His Leu Tyr Thr Phe His Ala Arg Phe Gly Glu Val Arg Pro Tyr Phe
    450                 455                 460

Tyr Leu Gly Gly Ala Arg Gly Ala Gly Pro Pro Glu Pro Leu Arg Ile
465                 470                 475                 480

Cys Pro Leu His Ile Ser Val Lys Glu Glu Leu Asp Gly
                485                 490

<210> 5
<211> 4339
<212> DNA
<213> Homo sapiens

<400> 5
   ctcgggtcgc gggcgttggc agccgggcgg gtgggagggg ccggagcaaa agttccgggc 60
   gcccgagccg gctgctcgtg ccatggagcg gagtcccgac gtgtcccccg ggccttcccg 120
   ctccttcaag gaggagttgc tctgcgccgt ctgctacgac cccttccgcg acgcagtcac 180
   tctgcgctgc ggccacaact tctgccgcgg gtgcgtgagc cgctgctggg aggtgcaggt 240
   gtcgcccacc tgcccagtgt gcaaagaccg cgcgtcaccc gccgacctgc gcaccaacca 300
   caccctcaac aacctggtgg agaagctgct gcgcgaggag gccgagggcg cgcgctggac 360
   cagctaccgc ttctcgcgtg tctgccgcct gcaccgcgga cagctcagcc tcttctgcct 420
   cgaggacaag gagctgctgt gctgctcctg ccaggccgac ccccgacacc aggggcaccg 480
   cgtgcagccg gtgaaggaca ctgcccacga ctttcgggtg aggagcctga tagccgaaga 540
   gagaaggaac tttttaccaa ctcaccagtg gatagtgaca aagacaaggc tccaaaccag 600
   ctctcctaac cttcagtcca ggaggcaagg ccaagtgcag gaacatggag catgcactgc 660
   gggagaaggc caaggccttc tgggccatgc ggcgctccta tgaggccatc gccaagcaca 720
   atcaggtgga ggctgcatgg ctggaaggcc ggatccggca ggagtttgat aagcttcgcg 780
   agttcttgag agtggaggag caggccattc tggatgccat ggccgaggag acaaggcaga 840
   agcaacttct ggccgacgag aagatgaagc agctcacaga ggagacggag gtgctggcac 900
   atgagatcga gcggctgcag atggagatga aggaggacga cgtttctttt ctcatgaaac 960
   acaagagccg aaaacgccga ctcttctgca ccatggagcc agagccagtc cagcccggca 1020
   tgcttatcga tgtctgcaag tacctgggct ccctgcagta ccgcgtctgg aagaagatgc 1080
   ttgcatctgt ggaatctgta cccttcagct ttgaccccaa caccgcagct ggctggctct 1140
   ccgtgtctga cgacctcacc agcgtcacca accatggcta ccgcgtgcag gtggagaacc 1200
   cggaacgctt ctcctcggcg ccctgcctgc tgggctcccg tgtcttctca cagggctcgc 1260
   acgcctggga ggtggccctt ggggggctgc agagctggag ggtgggcgtg gtacgtgtgc 1320
   gccaggactc gggcgctgag ggccactcac acagctgcta ccacgacaca cgctcgggct 1380
   tctggtatgt ctgccgcacg cagggcgtgg aggggaccca ctgcgtgacc tcggacccag 1440
   ccacgtcgcc cctggtcctg gccatcccac gccgcctgcg tgtggagctg gagtgtgagg 1500
   agggcgagct gtctttctat gacgcggagc gccactgcca cctgtacacc ttccacgccc 1560
   gctttgggga ggttcgcccc tacttctacc tggggggtgc acggggcgcc gggcctccag 1620
   agcctttgcg catctgcccc ttgcacatca gtgtcaagga agaactggat ggctgagctg 1680
   gcccggggct gccccggtct tgtgccacag cactgttttc tttctgccct cttcctaatg 1740
   cccacactgc ttgggcacta ttgcgcccct gcctccttgc caggctcttc ctcctgtcct 1800
   gcctggtcct tttccatgac tccaggctgt gcctctctcc atgtttggtc ccttctgtgc 1860
   ccatggtcag gagctattcg ggtggcacct cgctggccag gctctcccga gtcgtggcac 1920
   ctccacaatg tgaattttct gaatccctat tccaggattt ctgggaataa tgtttacttc 1980
   tagaatgggc ctgttgtaaa ccatctcatc gaggtgtggt aaagccattg gatgaggagg 2040
   ggactgccat ggaaaggaga gtttgttact tacggttctg agaggagggg ccacatagga 2100
   aagccccacg gtgggtcaga aggcggaagg agggagggga acgtgtgggc aagagacttc 2160
   ctctggtttc ctcaggagga aatgggcaag gcagagtaag caggggagac aggtttaagg 2220
   gtagctggct tgagtaattt cagtggctct caggataggg gctgcccttt ttgtctgata 2280
   cctggccccg ggatagtcag gacaggtgaa tgttggcctg gggtgtgaca gccctgggag 2340
   agccatgtga aggaggcagc tggcgccatc gctccggatt agttggtttc cataggaaag 2400
   gcatgctttc agccagatgc ttgccatctc tagggattgg gggattggct agcctgggag 2460
   gatcagtctg tccaggtcag cgaggcccca gataccagag catcaagagt acaggaaata 2520
   cagttaatgc agggcctctg tgtggctgga tcctccgtct ccatcagatc agctctgatt 2580
   gatctattct tgcacgattt cctctgaaca cagggttcca gagtacttaa acacaacatt 2640
   ttttaaatcg tgatttcggc ctatttcctt gccaggcctg tttccccacc aggaaatgag 2700
   ataggaggac tggatgagga tgtcctgtta tagttgctgt ggaggaagtt cctctggtta 2760
   attctcatca gcgtctgcag aaaagaagga aagagggcac ccttttcagt tgggaagaaa 2820
   ggagaggggt ggcgccatgg acgtggccct aaacgctgtg ggagagggaa gaggaggctg 2880
   ggcctcgctg ccctcttgtc tctgctgact tcagcctggt catgcttgct ctgccacttg 2940
   cgatttcatc cctaatttct tcctccacca tgcctgcaga cttttccctg ggcttgtttt 3000
   ttctcgcaca tctctgaaga gtttttaatc ttcagctcat catgtcccag gaagtggcat 3060
   cataaaagga aatatttttt tttcctagga gcagtgttaa aatctgggtc acattcctga 3120
   ccaaggacag catcctgcct tttggccatc cccttcagtt cacaaaagct gacattttaa 3180
   acaaatcatg actcacacgt attaattggt tataaatatg ttgtgtacac tggttagata 3240
   aaacttaagg ccacaaggag ggcccaggta ggcgatgtca gtgtgtgaag gggctggatt 3300
   gggcgtggtg aggatgttgg caaaccagtg catgcacctg gttggaagat gctcagcctc 3360
   acaaaagctc caagcccttt gggagccaaa gtgtctgaga gtgtgaccct ctcctgtaaa 3420
   gtatttatcc cacccattaa tataatttct gtataataaa cttgacctga aattatttca 3480
   ttctttatat taaactttta aaaatgtttt ttattttcac cttagatatg ggaagagttt 3540
   tttttttttt ttttttttta acaggataac ttgagcaggc taggcctctt aaaaaaaaat 3600
   ttgagctaaa actcattttt cttttggcat tttcttttca atgttcttat aagcaaagtt 3660
   catccatgtt gtagcatgtg ttcaacttta ttttttcatc gggtaatatt ccattgtatg 3720
   gaatggtagt actacatttt atttatcatg catcgattgg tggacatttg gatcgtttct 3780
   acttcttgac tattatacat aatgctgcta ggaacttttg tgtatgagtt tttgtgtgga 3840
   catatgtttt catttctctt tggtatatgc ctgggagcag atttgctgga tcatatgaaa 3900
   ctctatttaa cccttgaggg actcccaaac tgttttccaa tgtggtgaca caattttata 3960
   tcccatcaac agggcatgag ggttctgatg actccacatc cctcagtgct ttttattatc 4020
   tatcttttaa cttagccatc ctagtagggg taatgtggca tctcattgtg attttggttt 4080
   gcatttccct gatggcgaat gatattgagc atcttttcat gagcttattg gccatttgca 4140
   tatcttctta ggacagctat ctttagatca cttgctcatt ttttaattgg gttatttgtc 4200
   tttttattat tgagttgtaa gagtcctttt atagcctggc acaagtccct ttaactggta 4260
   tatgattata aaatttttct ccgtgagctg tttcatttcc ttgatgatgt cctttgaaat 4320
   actaaagctt ttaattttg 4339

<210> 6

<211> 206
<212> PRT
<213> Homo sapiens

<400> 6
Met Glu Arg Ser Pro Asp Val Ser Pro Gly Pro Ser Arg Ser Phe Lys
1               5                   10                  15

Glu Glu Leu Leu Cys Ala Val Cys Tyr Asp Pro Phe Arg Asp Ala Val
            20                  25                  30

Thr Leu Arg Cys Gly His Asn Phe Cys Arg Gly Cys Val Ser Arg Cys
        35                  40                  45

Trp Glu Val Gln Val Ser Pro Thr Cys Pro Val Cys Lys Asp Arg Ala
    50                  55                  60

Ser Pro Ala Asp Leu Arg Thr Asn His Thr Leu Asn Asn Leu Val Glu
65                  70                  75                  80

Lys Leu Leu Arg Glu Glu Ala Glu Gly Ala Arg Trp Thr Ser Tyr Arg
                85                  90                  95

Phe Ser Arg Val Cys Arg Leu His Arg Gly Gln Leu Ser Leu Phe Cys
            100                 105                 110

Leu Glu Asp Lys Glu Leu Leu Cys Cys Ser Cys Gln Ala Asp Pro Arg
        115                 120                 125

His Gln Gly His Arg Val Gln Pro Val Lys Asp Thr Ala His Asp Phe
    130                 135                 140

Arg Val Arg Ser Leu Ile Ala Glu Glu Arg Arg Asn Phe Leu Pro Thr
145                 150                 155                 160

His Gln Trp Ile Val Thr Lys Thr Arg Leu Gln Thr Ser Ser Pro Asn
                165                 170                 175

Leu Gln Ser Arg Arg Gln Gly Gln Val Gln Glu His Gly Ala Cys Thr
            180                 185                 190

Ala Gly Glu Gly Gln Gly Leu Leu Gly His Ala Ala Leu Leu
        195                 200                 205


<210> 7
<211> 192
<212> PRT
<213> Mus musculus

<400> 7
Met Glu Pro Gly Pro Ser Val Ser Pro Gly Pro Ser Arg Ser Phe Lys
1               5                   10                  15

Glu Glu Leu Leu Cys Ala Val Cys Tyr Asp Pro Phe Arg Asp Ala Val
            20                  25                  30

Thr Leu Arg Cys Gly His Asn Phe Cys Arg Arg Cys Val Ser Gly Cys
        35                  40                  45

Trp Glu Val Gln Thr Thr Pro Ser Cys Pro Val Cys Lys Glu Arg Ala
    50                  55                  60

Val Pro Gly Glu Leu Arg Thr Asn His Thr Leu Asn Asn Leu Val Glu
65                  70                  75                  80

Thr Leu Leu Arg Glu Glu Ala Glu Gly Ala Arg Trp Thr Gly Arg Arg
                85                  90                  95

Ser Pro Arg Pro Cys Arg Ala His Arg Ala Pro Leu Thr Leu Phe Cys
            100                 105                 110

Leu Glu Asp Lys Glu Leu Leu Cys Cys Ala Cys Gln Ala Asp Ala Arg
        115                 120                 125

His Gln Glu His Arg Val Gln Pro Ile Lys Asp Thr Ala Gln Asp Phe
    130                 135                 140

Arg Ala Lys Cys Lys Asn Met Glu His Val Leu Arg Glu Lys Ala Lys
145                 150                 155                 160

Ala Phe Trp Ala Leu Arg Arg Thr Tyr Glu Ala Ile Ala Lys His Asn
                165                 170                 175

Glu Val Gln Thr Thr Trp Leu Glu Gly Arg Ile Arg Asp Glu Phe Asp
            180                 185                 190

<210> 8
<211> 309
<212> PRT
<213> Mus musculus

<400> 8
Lys Leu Arg Asp Phe Leu Arg Val Glu Glu Gln Ala Thr Leu Asp Ala
1               5                   10                  15

Met Lys Glu Glu Ser Arg Lys Lys His Leu Gln Ala Glu Glu Lys Met
            20                  25                  30

Lys Gln Leu Ala Glu Gln Thr Glu Ala Leu Ala Arg Glu Ile Glu Arg
        35                  40                  45

Leu Gln Met Glu Met Lys Glu Asp Asp Met Thr Phe Leu Met Lys His
    50                  55                  60

Lys Ser Arg Lys Arg Arg Leu Phe Cys Thr Val Glu Pro Ala Pro Leu
65                  70                  75                  80

Gln Pro Gly Leu Leu Met Asp Ala Cys Lys Tyr Leu Glu Ser Leu Gln
                85                  90                  95

Tyr Arg Val Trp Lys Lys Met Leu Gly Ser Val Glu Ser Val Pro Phe
            100                 105                 110

Ser Leu Asp Pro Asn Thr Ala Ala Gly Trp Leu Lys Val Ala Asp Asp
        115                 120                 125

Leu Thr Ser Val Ile Asn His Gly Tyr Arg Val Gln Val Glu Asn Pro
    130                 135                 140

Glu Arg Phe Ser Ser Ala Pro Cys Leu Leu Gly Ser Gln Val Phe Ser
145                 150                 155                 160

Lys Gly Ser His Ser Trp Glu Val Asp Val Gly Gly Leu Pro Thr Trp
                165                 170                 175

Arg Val Gly Val Val Arg Val Gln Ala His Ala Gln Ala Gln Ala Gln
            180                 185                 190

Ala Asp Val Gly Gly Glu Gly His Ser His Ser Cys Tyr His Asp Thr
        195                 200                 205

Arg Ser Gly Phe Trp Tyr Leu Cys Arg Thr Gln Gly Val Asp Gly Asp
    210                 215                 220

His Cys Met Thr Ser Asp Thr Ala Thr Ala Pro Leu Val Gln Ala Met
225                 230                 235                 240

Pro Arg Arg Leu Arg Val Glu Leu Glu Cys Glu Glu Gly Glu Leu Ser
                245                 250                 255

Phe Tyr Asp Ser Glu Arg His Cys His Leu Tyr Thr Phe His Ala His
            260                 265                 270

Phe Gly Glu Val Arg Pro Tyr Phe Tyr Leu Gly Ala Ser Arg Gly Asp
        275                 280                 285

Gly Pro Pro Glu Pro Leu Arg Ile Cys His Leu Arg Val Ser Ile Lys
    290                 295                 300

Glu Glu Leu Asp Ile
305